# EUROPÄISCHE PATENTSCHRIFT

(11) **EP 0 934 326 B1**
(45) Veröffentlichungstag und Bekanntmachung des Hinweises auf die Patenterteilung: **03.05.2006**
(21) Anmeldenummer: 97946755.2
(22) Anmeldetag: 22.10.1997
(51) Int. Cl.: C07F 9/6509, A61K 31/675

(54) **SUBSTITUIERTE AMINOALKANPHOSPHONSÄUREN**
SUBSTITUTED AMINOALKANE PHOSPHONIC ACIDS
ACIDES AMINOALCANOPHOSPHONIQUES SUBSTITUES

(30) Priorität: 24.10.1996 CH 262196
(43) Veröffentlichungstag der Anmeldung: 11.08.1999
(73) Patentinhaber: Novartis AG, 4056 Basel (CH); Novartis Pharma GmbH, 1230 Wien (AT)
(72) Erfinder: ACKLIN, Pierre, CH-4127 Birsfelden (CH); ALLGEIER, Hans, D-79541 Lörrach (DE); AUBERSON, Yves, CH-4123 Allschwil (CH); OFNER, Silvio, CH-4142 Münchenstein (CH); VEENSTRA, Siem, Jacob, D-79540 Lörrach (DE)
(74) Vertreter: de Weerd, Petrus G.W.
(86) Internationale Anmeldenummer: PCT/EP1997/005843
(87) Internationale Veröffentlichungsnummer: WO 1998/017672

(56) Entgegenhaltungen:
- WO-A-94/25469
- WO-A-97/03079
- WO-A-97/08155

## Beschreibung

Die Erfindung betrifft die Verbindungen
N-Acetyl-N-(7-brom-2,3-dioxo-1,2,3,4-tetrahydrochinoxalin-5-ylmethyl)-aminomethylphosphonsäure;
N-Acetyl-N-(7-chlor-2,3-dioxo-1,2,3,4-tetrahydrochinoxalin-5-ylmethyl)-aminomethylphosphonsäure;
N-Acetyl-N-(7-fluor-2,3-dioxo-1,2,3,4-tetrahydrochinoxalin-5-ylmethyl)-aminomethylphosphonsäure;
N-(2,3-Dioxo-7-nitro-1,2,3,4-tetrahydrochinoxalin-5-ylmethyl)-α-(ethytamino)-ethylphosphonsäure;
N-(7-Chlor-2,3-dioxo-1,2,3,4-tetrahydrochinoxalin-5-ylmethyl)-α-(methylamino)-ethylphosphonsäure;
N-(7-Fluor-2,3-dioxo-1,2,3,4-tetrahydrochinoxalin-5-ylmethyl)-α-(ethylarnino)-ethylphosphonsäure;
N-(2,3-Dioxo-7-nitro-1,2,3,4-tetrahydrochinoxalin-5-ylmethyl)-α-(methylamino)-ethylphosphonsäure;
N-(2,3-Dioxo-7-nitro-1,2,3,4-t etrahydrochinoxalin-5-ylmethyl)-β-amino-propylphosphonsäure;
N-(2,3-Dioxo-7-nitro-1,2.3,4-tetrahydrochinoxalin-5-ylmethyl)-2-amino-phenylphosphonsäure;
N-(7-Brom-2,3-dioxo-1,2,3,4-tetrahydrochinoxalin-5-ylmethyl)-α-aminobenzylphosphonsäure;
N-(7-Bromo-2,3-dioxo-1,2,3,4-tetrahydrochinoxalin-5-ylmethyl)-α-amino-3-methylbutylphosphonsäure;N-(7-Bromo-2,3-dioxo-1,2,3,4-tetrahydrochinoxalin-5-ylmethyl)-α-amino-β-benzyloxyethylphosphonsäure;
N-(7-Bromo-2,3-dioxo-1,2,3,4-tetrahydrochinoxatin-5-ylmethyl)-α-amino-propylphosphonsäure;
N-(2,3-Dioxo-7-nitro-1,2,3,4-tetrahydrochinoxalin-5-ylmethyl)-4-amino-benzylphosphonsäurediethylesterN-(2,3-Dioxo-7-nitro-1,2,3,4-tetrahydrochinoxalin-5-ylmethyl)-α-amino-(3-hydroxybenzyl)-phosphonsäure;
N-(2,3-Dioxo-7-nitro-1,2,3,4-tetrahydrochinoxalin-5-ylmethyl)-α-amino-(isopropyl)-phosphonsäure;
N-(2,3-Dioxo-7-nitro-1,2,3,4-tetrahydrochinoxalin-5-ylmethyl)-4-amino-benzylphosphonsäuretrans-2-[N-(2,3-Dioxo-7-nitro-1,2,3,4-tetrahydrochinoxalin-5-ylmethyl)-amino]-cyclopropylphosphonsäure;
N-(2,3-Dioxo-7-nitro-1,2,3,4-tetrahydrochinoxalin-5-ylmethyl)-α-amino-(3,4,5-trimethoxybenzyl)-phosphonsäure;
N-(2,3-Dioxo-7-nitro-1,2,3,4-tetrahydrochinoxalin-5-ylmethyl)-α-amino-cyclohexylmethylphosphonsäure;
N-(2,3-Dioxo-7-nitro-1,2,3,4-tetrahydrochinoxalin-5-ylmethyl)-α-amino-*n*-butyl-phosphonsäure;
N-(2,3-Dioxo-7-nitro-1,2,3,4-tetrahydrochinoxalin-5-ylmethyl)-α-amino-3-methylbutyl-phosphonsäure;
N-(2,3-Dioxo-7-nitro-1,2,3,4-tetrahydrochinoxalin-5-ylmethyl)-α-amino-benzylphosphonsäure;
N-(2,3-Dioxo-7-nitro-1,2,3,4-tetrahydrochinoxalin-5-ylmethyl)-α-amino-(3-thienyl)-methylphosphonsäure;
N-(2,3-Dioxo-7-nitro-1,2,3,4-tetrahydrochinoxalin-5-ylmethyl)-α-amino-(4-methoxycarbonylbenzyl)-phosphonsäure;
N-(2,3-Dioxo-7-nitro-1,2,3,4-tetrahydrochinoxalin-5-ylmethyl)-α-amino-(3-nitrobenzyl)-phosphonsäure;
-(2,3-Dioxo-7-nitro-1,2,3,4-tetrahydrochinoxalin-5-ylmethyl)-α-amino-(2-hydroxy-3-methoxybenzyl)-phosphonsäure;
N-(7-Chlor-2,3-dioxo-1,2,3,4-tetrahydrochinoxalin-5-ylmethyl)-2-aminophenylphosphonsäure;
N-(7-Nitro-2,3-dioxo-1,2,3,4-tetrahydrochinoxalin-5-ylmethyl)-2-aminophenylphosphonsäure;
N-(7-Chlor-2,3-dioxo-1,2,3,4-tetrahydrochinoxalin-5-ylmethyl)-α-amino-ethylphosphonsäure;
N-(7-Fluor-2,3-dioxo-1,2,3,4-telrahydrochinoxalin-5-ylmethyl)-α-amino-ethylphosphonsäure;
P-Benzyl N-(2,3-dioxo-7-nitro-1,2,3,4-teirahydrochinoxalin-5-ylmethyl)-aminomethylphosphinsäure;
P-Methyl-N-(2,3-dioxo-7-nitro-1,2,3,4-tetrahydrochinoxalin-5-ylmethyl)-α-aminoethylphosphinsäure;
P-Benzyl-(7-nitro-2,3-dioxo-1,2,3,4-tetrahydrochinoxalin-5-yl)-aminomethanphosphinsäure;
P-Methyl-(7-nitro-2,3-dioxo-1,2,3,4-tetrahydrochinoxalin-5-yl)-aminomethanphosphinsäure;
N-(7-Nitro-2,3-dioxo-1,2,3,4-tetrahydrochinoxalin-5-ylmethyl)-4-phosphono-buttersäureamid;
N-(7-Nitro-2,3-dioxo-1,2,3,4-tetrahydrochinoxalin-5-ylmethyl)-2-phosphono-essigsäureamid;
N-(7-Nitro-2,3-dioxo-1,2,3,4-tetrahydrochinoxalin-5-ylmethyl)-3-phosphonopropensäureamid;
N-(7-Nitro-2,3-dioxo-1,2,3,4-tetrahydrochinoxalin-5-ylmethyl)-2-phosphono-indan-2-carbonsäureamid;
N-Benzyl-N-(2,3-dioxo-7-nitro-1,2,3,4-tetrahydrochinoxalin-5-ylmethyl)-amino-methylphosphonsäure;
N-Benzyl-N-(7-brom-2,3-dioxo-1,2,3,4-tetrahydrochinoxalin-5-ylmethyl)-amino-methylphosphonsäure;
N-Benzyl-N-(7-chlor-2,3-dioxo-1,2.3,4-tetrahydroch'snoxalin-5-Ylmethyl)-amino-methylphosphonsäure;
N-Benzyl-N-(2,3-dioxo-7-fluor-1,2,3,4-tetrahydrochinoxalin-5-ylmethyl)-amino-methylphosphonsäure;
N-Benzyl-N-(2,3-dioxo-7-nitro-1,2,3,4-tetrahydrochinoxalin-5-ylmethyl)-α-aminoethanphosphonsäure;
N-(2,3-Dioxo-7-fluor-1,2,3,4-tetrahydrochinoxalin-5-ylmethyl)-α-aminoethylphosphonsäure;
N-(2,3-Dioxo-7-nitro-1,2,3,4-tetrahydro-chinoxalin-5-ylmethyl)-α-amino-propylphosphonsäure;
N-(2,3-Dioxo-7-nitro-1,2,3,4-tetrahydro-chinoxalin-5-ylmethyl)-α-amino-(tetrahydropyran-4-yl)-phosphonsäure;
N-(2,3-Dioxo-7-nitro-1,2,3,4-tetrahydro-chinoxalin-5-ylmethyl)-α-amino-(piperidin-4-yl)-phosphonsäure;
N-(2,3-Dioxo-7-nitro-1,2,3,4-tetrahydro-chinoxalin-5-ylmethyl)-α-amino-(furan-2-ylmethyl)-phosphonsäure;
N-(2,3-Dioxo-7-nitro-1,2,3,4-tetrahydro-chinoxalin-5-ylmethyl)-α-amino-(2-methoxy)-ethylphosphonsäure;
N-(2,3-Dioxo-7-nitro-1,2,3,4-tetrahydro-chinoxalin-5-ylmethyl)-2-amino-2-cyclohexyl-ethylphosphonsäure;
N-(2,3-Dioxo-7-nitro-1,2,3,4-tetrahydro-chinoxalin-5-ylmethyl)-β-amino-isopropyl-phosphonsäure;
N-(2,3-Dioxo-7-nitro-1,2,3,4-tetrahydro-chinoxalin-5-ylmethyl)-2-amino-cyclohexyl-phosphonsäure;
N-(2,3-Dioxo-7-nitro-1,2,3,4-tetrahydro-chinoxalin-5-ylmethyl)-2-amino-2-methyl-propylphosphonsäure;
N-(2,3-Dioxo-7-nitro-1,2,3,4-tetrahydro-chinoxalin-5-ylmethyl)-2-amino-butyl-phosphonsäure;
N-(2,3-Dioxo-7-nitro-1,2,3,4-tetrahydro-chinoxalin-5-ylmethyl)-2-amino-heptyl-phosphonsäure;
N-(2,3-Dioxo-7-nitro-1,2,3,4-tetrahydro-chinoxalin-5-ylmethyl)-2-amino-3-phenyloxy-propylphosphonsäure;
N-(2,3-Dioxo-7-nitro-1,2,3,4-tetrahydro-chinoxalin-5-ylmethyl)-2-amino-2-*p*-tolyl-ethylphosphonsäure;
N-(2,3-Dioxo-7-nitro-1,2,3,4-tetrahydro-chinoxalin-5-ylmethyl)-2-amino-2-(2-methoxy-phenyl)-ethylphosphonsäure;
N-(2,3-Dioxo-7-nitro-1,2,3,4-tetrahydro-chinoxalin-5-ylmethyl)-2-amino-2-(4-fluor-phenyl)-ethylphosphonsäure;
N-(2,3-Dioxo-7-nitro-1,2,3,4-tetrahydro-chinoxalin-5-ylmethyl)-2-amino-2-phenyl-ethylphosphonsäure;
N-(2,3-Dioxo-7-nitro-1,2,3,4-tetrahydro-chinoxalin-5-ylrnethyt)-2-amino-1-(furan-2-yl)-ethylphosphonsäure;
N-(2,3-Dioxo-7-nitro-1,2,3,4-tetrahydro-chinoxalin-5-ylmethyl)-2-amino-1-(4-fluor-phenyl)-ethylphosphonsäure;
N-(2,3-Dioxo-7-nitro-1,2,3,4-letrahydro-chinoxalin-5-ylmethyl)-2-amino-1-(4-methoxy-phenyl)-ethylphosphonsäure;
N-(2,3-Dioxo-7-nitro-1,2,3,4-tetrahydro-chinoxalin-5-ylmethyl)-2-amino-1-(3-methoxy-phenyt)-ethylphosphonsäure;
N-(2,3-Dioxo-7-nitro-1,2,3,4-tetrahydro-chinoxalin-5-ylmethyl)-2-amino-1-(2-chlor-phenyl)-ethylphosphonsäure;
N-(2,3-Dioxo-7-nitro-1,2,3,4-tetrahydro-chinoxatin-5-ylmethyl)-2-amino-1-(4-tolyl)-ethylphosphonsäure:
N-Benzyl-N-(7-brom-2,3-dioxo-1,2,3,4-tetrahydrochinoxalin-5-ylmethyl)-α-amino-ethylphosphonsäure;
N-Benzyl-N-(7-chlor-2,3-dioxo-1,2,3,4-tetrahydrochinoxalin-5-ylmethyl)-α-amino-ethylphosphonsäure und
N-Benzyl-N-(2,3-dioxo-7-fluor-1,2,3,4-tetrahydrochinoxalin-5-ylmethyl)-α-amino-ethylphosphonsäure;
und deren Salze. ("Verbindungen der Formel I")

Die Erfindung betrifft ferner
- eine der vorstehend genannten Verbindungen in freier Form oder in einer pharmazeutisch verwendbaren Salzform zur Anwendung in einem Verfahrne zur therapeutischen Behandlung des menschlichen oder tierischen Körpers
- pharmazeutische Präparate , enthaltend eine der vorstehend genannten Verbindungen in freier Form oder in einer pharmazeutisch verwendbaren Salzform neben üblichen Hilfs- und Trägerstoffen
- die Verwendung einer der vorstehend genannten Verbindungen in freier Form oder in einer pharmazeutisch verwendbaren Salzform zur Herstellung eines Arzneimittels zur Behandlung von pathologischen Zuständen, die auf die Blockierung von excitadorischen Aminosäurerezeptoren ansprechen.
- die Verwendung einer der vorstehend genannten Verbindungen in freier Form oder in einer pharmazeutisch verwendbaren Salzform zur Herstellung eines Arzneimittels zur Behandlung von neurodegenerativen Erkrankungen, ischämischen Hirnerkrankungen, Gefäss- und Muskelspasmen, Konvulsionen, Angst und Schmerzzuständen; insbesondere von Epilepsie.

WO94/25469 offenbart Chinoxalidone - Derivate, deren Herstellung und Verwendung als Arzneimittel.

WO 97/08155 offenbart 2,3-dioxo-1,2,3,4 tetrahydroquioxylinyl - Derivate, deren Herstellung und Verwendung als Arzneimittel.

Die Verbindungen der Formel I können Salze mit Basen bilden. Verbindungen der Formel 1 mit basischen Gruppen können ferner Säureadditionssalze und innere Salze bilden.

Salze von Verbindungen der Formel I mit Basen sind beispielsweise deren Salze mit pharmazeutisch verwendbaren Basen, wie nicht-toxische, von Metallen der Gruppen la, Ib, IIa und IIb abgeleitete Metallsalze, z.B. Alkalimetall-, insbesondere Natrium- oder Kaliumsalze, Erdalkalimetall-, insbesondere Calcium- oder Magnesiumsalze, ebenso Ammoniumsalze mit Ammoniak oder organischen Aminen oder quaternären Ammoniumbasen, wie gegebenenfalls C-hydroxylierten aliphatischen Aminen, insbesondere Mono-, Di- oder Triniederalkylaminen, z.B. Methyl-, Ethyl- oder Diethylamin, Mono-, Di- oder Tri-(hydroxyniederalkyl)aminen, wie Ethanolamin, Diethanolamin oder Triethanolamin, Tris-(hydroxymethyl)Naphthylreste oder 2-Hydroxytertiärbutylamin, oder N-(Hydroxyniederalkyl)-N,N-diniederalkyl-aminen bzw. N-(Polyhydroxyniederalkyl)-N-niederalkylaminen, wie 2-(Dimethylamino)ethanol oder D-Glucamin oder Cholin, oder quaternären aliphatischen Ammoniumhydroxiden, z.B. Tetrabutylammoniumhydroxid.

Säureadditionssalze von Verbindungen der Formel 1 sind beispielsweise deren pharmazeutisch verwendbare Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, Salze mit geeigneten Carbonsäuren, wie gegebenenfalls hydroxylierten Niederalkansäuren, z.B. Essigsäure, Glykolsäure, Propionsäure, Milchsäure oder Pivalinsäure, gegebenenfalls hydroxylierten und/oder durch Oxo substituierten Niederalkandicarbonsäuren, z.B. Oxalsäure, Bernsteinsäure, Fumarsäure, Maleinsäure, Weinsäure, Zitronensäure, Brenztraubensäure, Äpfelsäure, Ascorbinsäure, ferner mit aromatischen, heteroaromatischen oder araliphatischen Carbonsäuren, wie Benzoesäure, Nicotinsäure oder Mandelsäure, und Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate).

Umfasst sind sowohl vollständige als auch partielle Salze, d.h. Salze mit 1, 2 oder 3, vorzugsweise 2, Äquivalenten Base pro Mol Säure der Formel I bzw. Salze mit 1, 2 oder 3 Äquivalenten, vorzugsweise 1 Äquivalent, Säure pro Mol Base der Formel 1.

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die deshalb bevorzugt sind.

Die Verbindungen der Formel 1 weisen wertvolle pharmakologische Eigenschaften auf. Sie besitzen eine hohe Bindungsaffinität gegenüber excitatorischen Aminosäurerezeptoren, wie gegenüber AMPA-Rezeptoren, NMDA(Kainate)-Rezeptoren und/oder Glycinbindungsstellen von NMDA-Rezeptoren. Die Affinität zu den genannten Rezeptoren ist je nach Struktur global oder selektiv. Ausgewählte Verbindungen der Formel I weisen insbesondere eine starke Affinität zu AMPA- und/oder NMDA(Kainate)-Bindungsstellen und eine weniger starke Affinität zu Glycinbindungsstellen des NMDA-Rezeptors auf.

Das Bindungsvermögen der erfindungsgemäss bereitgestellten Verbindungen und ihrer Salze kann *in vitro* radiographisch an Himmembranen von Ratten (Maus, Ratte) anhand der Verdrängung von [³H]-AMPA, [³H]-Kainate, [³H]-DCKA (5,7-Dichlorkynurensäure) bzw. [³H]-MDL 105,510 nachgewiesen werden, wobei die für eine 50 %-ige Verdrängung erforderliche Konzentration (IC₅₀) bestimmt wird.

Zur Bestimmung der Bindungsaffinität zu AMPA-Rezeptoren kann beispielsweise die Radioreceptorassay-Versuchsanordnung von Honore T., Lauridsen J. und Krogsgaard-Larsen gemäss J. Neurochem. 38, 173-178 (1981) herangezogen werden, in der Verbindungen der Formel I IC₅₀-Werte von etwa 0.05 bis etwa 5 µM zeigen. Die Bindungsaffinität zu Kainate-Rezeptoren kann beispielsweise mittels der Radioreceptorassay-Versuchsanordnung von Simon J.R, Contrera J.F und Kuhn M.J, J. Neurochem 26, 141-147 (1975) gemessen werden, in der Verbindungen der Formel I IC₅₀₋Werte von etwa 0.5 bis etwa 5 µM zeigen.

Das Bindungsvermögen von Verbindungen der Formel I an Glycinbindungsstellen des NMDA-Rezeptors kann beispielsweise in der Radioreceptorassay-Versuchsanordnung nach Baron M.B, Siegel B.W. et al., Eur. J. Pharmacol., Molec. Pharmacol. Section 206, Seiten 149-154 (1991) und Canton T, Doble A. et al., J. Pharm. Pharmacol. 44, Seiten 812-816 (1992) an Rattenkortex- und Rattenhippocampusmembranen ermittelt werden. Der IC₅₀₋Wert von Verbindungen der Formel 1 liegt in dieser Versuchsanordnung im Bereich von etwa 0.005 bis etwa 5 µM.

Auf Grund dieser Eigenschaften weisen die Verbindungen der Formel ausgeprägte antikonvulsive Eigenschaften auf, die *in vivo,* beispielsweise an der Maus anhand ihrer ausgeprägten Schutzwirkung gegenüber durch Elektroschock oder Metrazol ausgelösten Konvulsionen bestimmt werden, wobei man z.B. das etablierte Elektroschock-Mausmodell bzw. das Mausmodell für Metrazol-induzierte Konvulsionen gemäss Schmutz et al., Naunyn-Schmiedeberg's Arch. Pharmacol. 342, 61-66 (1990), heranziehen kann.

Die Verbindungen der Formel I und ihre pharmazeutisch verwendbaren Salze sind somit vorzüglich geeignet zur prophylaktischen und therapeutischen Behandlung von pathologischen Zuständen, die auf Blockierung von excitatorischen Aminosäurerezeptoren, insbesondere Blockierung eines oder mehrerer der genannten Subtypen von excitatorischen Aminosäurerezeptoren, ansprechen, beispielsweise von neurodegenerativen Erkrankungen, wie von neurodegenerativen Erkrankungen im Gefolge von Schlaganfall, Hypoglykämie, Anoxie oder Hirnlähmungserscheinungen, von ischämischen Hirnerkrankungen, wie der cerebralen Ischämie, der cerebralen lschämie bei Herzchirurgie oder Herzstillstand, perinataler Aphyxie, epileptischer Anfälle, Chorea Huntington, Alzheimer'scher und Parkinson'scher Erkrankung, amyotroper Lateralsklerose, Rückenmark- und Hirntrauma sowie Vergiftungserscheinungen durch Neurotoxine oder Suchtmittelmissbrauch, und von ischämischen Erkrankungen des Auges, von Gefäss- und Muskelspasmen, wie von Migräne oder von lokaler oder generaler Spastizität, von Konvulsionen, wie der Epilepsie, und von Angst- und Schmerzzuständen, wie von Trigeminusneuralgien.

Die Erfindung betrifft namentlich die in den Beispielen genannten Verbindungen der Formel I und ihre Salze, Verfahren zu ihrer Herstellung, diese enthaltende pharmazeutische Präparate und ihre Verwendung als Arzneimittelwirkstoffe.

Das Verfahren zur Herstellung der Verbindungen der Formel I ist dadurch gekennzeichnet, dass man
aus einer Verbindungen der Formel 11 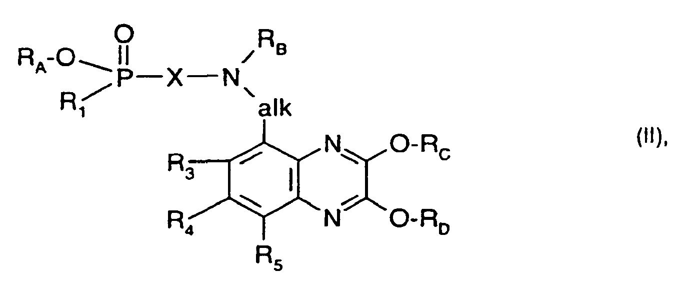 worin
R_{A} für Wasserstoff oder eine Hydroxyschutzgruppe steht, R_{B} eine Gruppe R₂ oder eine Aminoschutzgruppe darstellt und die Reste R_{C} und R_{D} gleiche oder verschiedene Hydroxyschutzgruppen bedeuten und R₁, X, R₂, alk, R₃, R₄ und R₅ die angegebenen Bedeutungen haben, die Hydroxyschutzgruppen R_{C} und R_{D} sowie eine gegebenenfalls vorhandene Hydroxyschutzgruppe R_{A} und eine gegebenenfalls vorhandene Aminoschutzgruppe R_{B} abspaltet
und gewünschtenfalls jeweils eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

Als Hydroxyschutzgruppen R_{A} kommen beispielsweise die für den intermediären Schutz von Phosphonogruppen üblichen Hydroxyschutzgruppen in Betracht, wie insbesondere Niederalkyl, z.B. Methyl, Ethyl oder Isopropyl, ferner gegebenenfalls substituierte Phenylniederalkyl-, wie Benzylgruppen, ebenso Triniederalkylsilyl, wie Trimethylsilyl, in Betracht. Hydroxyschutzgruppen R_{C} und/oder R_{D} sind beispielsweise die für den intermediären Schutz von Laktamgruppen üblichen Hydroxyschutzgruppen, insbesondere etherbildende Gruppen, wie Niederalkyl, vorzugsweise Methyl.

Geeignete Aminoschutzgruppen R_{B} sind beispielsweise die für den intermediären Schutz von Aminogruppen gebräuchlichen Schutzgruppen, wie von einem Halbester der Kohlensäure abgeleitete Acylgruppen, wie Niederalkoxycarbonyl oder gegebenenfalls substituierte Phenyloxy- oder Phenylniederalkoxycarbonylgruppen.

Die Abspaltung der genannten Schutzgruppen R_{C}, R_{D} und gegebenenfalls R_{A} und/oder R_{B} erfolgt in üblicher Weise, beispielsweise durch Säurebehandlung, wie Behandlung mit Salzsäure, beispielsweise 1 N- bis 4N-Salzsäure, etwa 20%-iger bis etwa 40%-iger Bromwasserstoffsäure in Essigsäure, oder Behandlung mit einem Triniederalkylhalogensilan, wie Trimethylbromsilan, oder einem Hexaniederalkyldisilazan, wie Hexamethyldisilazan, unter nachträglicher Zugabe eines Niederalkanols, wie Ethanol, welcher mit Silankomponente unter Freisetzung von Halogenwasserstoff reagiert. Alternativ kann man die Schutzgruppenabspaltung auch durch Behandlung mit einer Lewissäure, wie einem Triniederalkylhalogensilan, durchführen, wobei indes drastischere Reaktionsbedingungen, wie Temperaturen im Bereich von etwa 50° C bis etwa 110° C, erforderlich sein können.

Ausgangsstoffe der Formel II werden beispielsweise hergestellt, indem man in einer entsprechenden Verbindung der Formel III 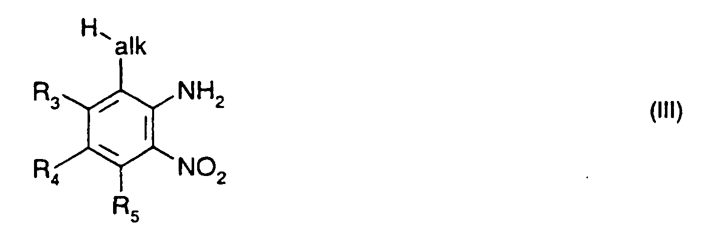 die Nitrogruppe in üblicher Weise, beispielsweise durch katalytische Hydrierung in Gegenwart von Palladium oder Raney-Nickel, zu Amino reduziert, das erhaltene Phenylen-1,2-diamin unter sauren Bedingungen, beispielsweise in Gegenwart von Salzsäure, mit Oxalsäure zum entsprechenden Chinoxalindion der Formel IV 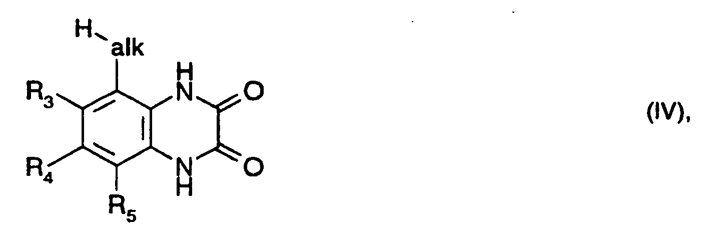 kondensiert, dieses durch Umsetzung mit einem Halogen Hal einführenden Halogenierungsmittel, beispielsweise Phosphoroxychlorid, in das entsprechende 2,3-Dihalogenchinoxalin der Formel V 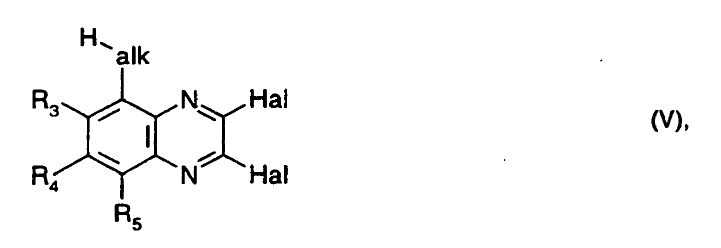 überführt, in diesem die Gruppen Hal in üblicher Weise, beispielsweise durch Umsetzung mit einem Alkalimetallniederalkanolat, wie Natriummethanolat, durch geschütztes Hydroxy -OR_{C} bzw. -OR_{D}, wie Niederalkoxy, insbesondere Methoxy, ersetzt, die erhaltene Verbindung der Formel VI 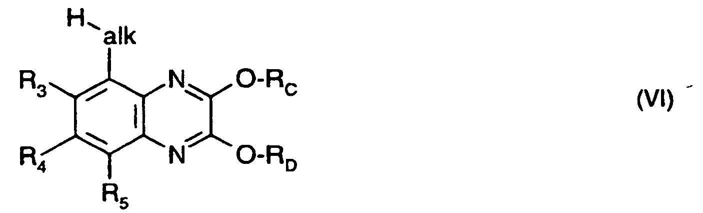 mit einem Halogen Hal einführenden Halogenierungsmittel, wie N-Bromsuccinimid in Gegenwart von Azo-isobutyronitril oder Dibenzoylperoxid, in der Seitenkette zur entsprechenden Verbindungen der Formel VII 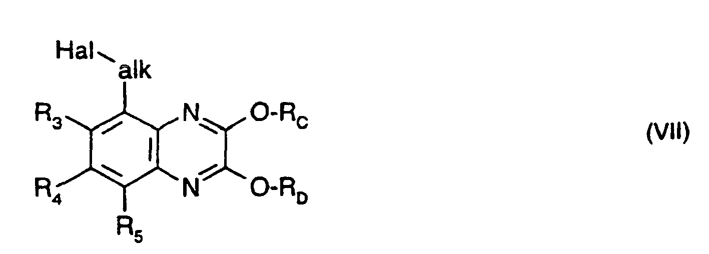 halogeniert und dieses durch Umsetzung mit einem Azid, wie Natriumazid, und anschliessende Reduktion, beispielsweise Behandlung mit Triphenylphosphin und Wasser, in die entsprechende Aminoverbindung der Formel VIII 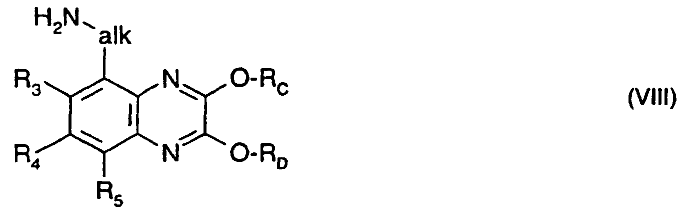 überführt, und dieses entweder mit einer Carbonylverbindung der Formel H-X=O (IX) und anschliessend in Gegenwart einer Base, wie eines Triniederalkylamins, mit einem Triniederalkylsilylether, wie dem Trimethylsilylether, einer Verbindungen der Formel X 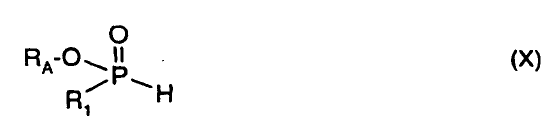 oder in Gegenwart von Natriumcyanoborhydrid direkt mit einer Verbindung der Formel XI 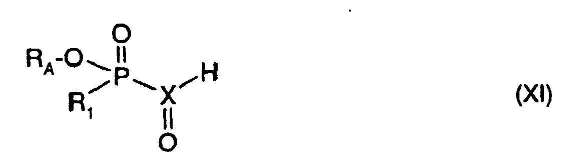 kondensiert. Diese Verfahrensvariante ist insbesondere geeignet für die Herstellung von Verbindungen der Formel 1, worin X einen zweiwertigen aliphatischen, cycloaliphatischaliphatischen, araliphatischen, heteroarylaliphatischen Rest bedeutet.

In einer für die Herstellung von Verbindungen der Formel I, worin X Niederalkyliden, insbesondere Methylen, darstellt, vorteilhaften Abwandlung dieser Verfahrensvariante setzt man das Amin der Formel X mit Formaldehyd oder einem Formaldehyd abgebenden Mittel in das entsprechende Triazinan der Formel XII 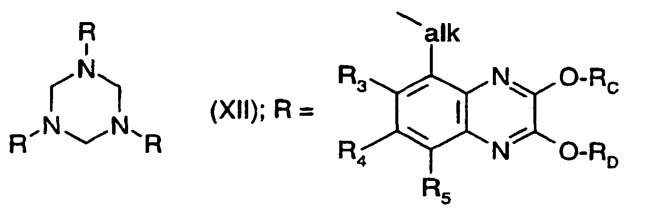 um und setzt dieses, beispielsweise in Gegenwart einer Base, wie eines Triniederalkylamins, mit einem Triniederalkylsilylether, wie dem Trimethylsilylether, der Verbindungen der Formel X um.

Zur Herstellung von Verbindungen der Formel II, worin X in α-Stellung zur Aminogruppe eine Oxogruppe trägt, setzt man die Aminverbindung VIII vorteilhaft in Gegenwart eines wasserbindenden Mittels, wie eines Carbodiimides, z.B. von N-(Dimethylaminopropyl)-N'-ethyl-carbodiimid-hydrochlorid, mit einer entsprechenden Carbonsäureverbindungen der Formel XIII 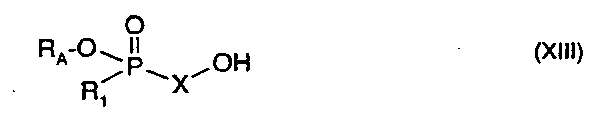 oder in Gegenwart eines basischen Kondensationsmittels mit einem reaktionsfähigen Derivat, wie einem Säurehalogenid oder reaktionsfähigen Ester, derselben um.

Zur Herstellung von Verbindungen der Formel II, worin X einen zweiwertigen cycloaliphatischen oder aromatischen Rest bedeutet, geht man vorteilhaft von Halogenverbindungen der Formel VII aus und kondensiert diese in üblicher Weise mit einer entsprechenden Aminkomponente der Formel XIV

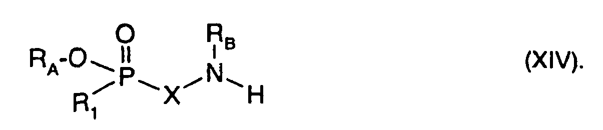

Verfahrensgemäss erhältliche Verbindungen können in üblicher Weise in andere Verbindungen der Formel überführt werden.

So kann man in Verbindungen der Formel 1, worin R₂ Wasserstoff bedeutet, in üblicher Weise aliphatische oder araliphatische Reste, wie Niederalkyl-, Niederalkenyl- oder Niederalkanoylreste, einführen, beispielsweise durch Umsetzung mit einem Niederalkylierungsmittel, wie einem Niederalkylhalogenid oder einem reaktionsfähigen Niederalkansäurederivat, wie einem Niederalkansäurechlorid, erforderlichenfalls in Gegenwart eines üblichen basischen Kondensationsmittels.

Ferner kann man veresterte oder amidierte Carboxygruppen enthaltende Verbindungen der Formel in üblicher Weise zu den entsprechenden Carbonsäuren hydrolysieren, bzw. freies Carboxy enthaltende Verbindungen der Formel 1 in üblicher Weise verestern oder amidieren.

Weiterhin kann man in erhaltenen Verbindungen der Formel 1 Cyano in üblicher Weise in Carbamoyl überführen.

Erhaltene Salze können in an sich bekannter Weise in die freien Verbindungen umgewandelt werden, z.B. durch Behandeln mit einer Base, wie einem Alkalimetallhydroxid, einem Metalicarbonat oder -hydrogencarbonat, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formel 1, einschliesslich ihrer Salze, können auch in Form von Hydraten erhalten werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Erhaltene Diastereomerengemische und Racematgemische können auf Grund der physikaüsch-chemischen Unterschiede der Bestandteile in bekannter Weise in die reinen Diastereomeren bzw. Racemate aufgetrennt werden, beispielsweise durch Chromatographie und/oder fraktionierte Kristallisation.

Erhaltene Racemate lassen sich ferner nach bekannten Methoden in die optischen , Antipoden zerlegen, beispielsweise durch Umkristallisation aus einem optisch aktiven Lösungsmittel, mit Hilfe von Mikroorganismen oder durch Umsetzung des erhaltenen Diastereomerengemisches bzw. Racemates mit einer optisch aktiven Hilfsverbindung, z.B. entsprechend der in Verbindungen der Formel 1 enthaltenen sauren, basischen oder funktionell abwandelbaren Gruppen mit einer optisch aktiven Säure, Base oder einem optisch aktiven Alkohol, in Gemische diastereomerer Salze bzw. funktioneller Derivate, wie Ester, Trennung derselben in die Diastereomeren, aus denen das jeweils gewünschte Enantiomere in der jeweils üblichen Weise freigesetzt werden kann. Dafür geeignete Basen, Säuren bzw. Alkohole sind beispielsweise optisch aktive Alkaloidbasen, wie Strychnin, Cinchonin oder Brucin, oder D- oder L-(1-Phenyl)ethylamin, 3-Pipecolin, Ephedrin, Amphetamin und ähnliche synthetisch zugängliche Basen, optisch aktive Carbon- oder Sulfonsäuren, wie Chinasäure oder D- oder L-Weinsäure, D- oder L-Di-o-toluylweinsäure, D- oder L-Äpfelsäure, D- oder L-Mandelsäure, oder D- oder L-Camphersulfonsäure, bzw. optisch aktive Alkohole, wie Borneol oder D- oder L-(1-Phenyl)ethanol.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeicheten Verbindungen der Formel 1 führende Ausgangsstoffauswahl, die Verfahren zu ihrer Herstellung und ihre Verwendung als Zwischenprodukte bilden ebenfalls einen Gegenstand der Erfindung.

Bei den erfindungsgemässen pharmazeutischen Präparaten, welche die erfindungsgemässen Verbindung oder pharmazeutisch verwendbare Salze davon enthalten, handelt es sich um solche zur enteralen, wie oralen, ferner rektalen, und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffes hängt von dem Alter und dem individuellen Zustand sowie von der Applikationsweise ab.

Die neuen pharmazeutischen Präparate enthalten z.B. von etwa 10 % bis etwa 80 %, vorzugsweise von etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragee-Kernen verarbeitet.

Geeignete Trägerstoffe sind insbesondere Füllstoffe, wie Zucker, z.B. Laktose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Traganth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliess-, Fliessregulier- und Schmiermittel, z.B. Kieselsäure, Talk, Stearinsäure oder Salze davon, wie Magnesium- oder Calciumstearat, und/oder Polyethylenglykol. Dragee-Kerne werden mit geeigneten, gegebenenfalls Magensaft-resistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragee-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulates, z.B. im Gemisch mit Füllstoffen, wie Laktose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wässerlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Dosierung des Wirkstoffes hängt von der Warmblüter-Spezies, dem Alter und dem individuellen Zustand sowie der Applikationsweise ab. Im Normalfall ist für einen etwa 75 kg schweren Patienten bei oraler Applikation eine ungefähre Tagesdosis von etwa 10 mg bis etwa 500 mg zu veranschlagen.

Die nachfolgen Beispiele dienen zur Illustration der Erfindung; Temperaturen sind in Celsiusgraden, Drucke in mbar angegeben.

### Beispiel 1: N-Acetyl-N-(7-brom-2,3-dioxo-1,2,3,4-tetrahydrochinoxalin-5-ylmethyl)-aminomethylphosphonsäure

Unter einer Stickstoffatmosphäre werden 400 mg (0.87 mMol) N-Acetyl-N-(7-brom-2,3-dimethoxy-chinoxalin-5-ylmethyl)-α-aminomethylphosphonsäuredimethylester in 5 ml absolutem Dichlormethan gelöst und bei Raumtemperatur mit 0.66 ml (5 mMol) Trimethylsilylbromid versetzt. Nach 16 Stunden Rühren bei Raumtemperatur werden 5 ml Ethanol zugegeben und weitere 6 Stunden bei Raumtemperatur gerührt. Anschliessend wird bis zur Trockene eingeengt. Der Rückstand wird in Essigester aufgenommen, mit Hexan versetzt bis zur leichten Trübung und über Nacht im Kühlschrank stehen gelassen. Der Niederschlag wird abfiltriert, am Hochvakuum getrocknet und zur weiteren Reinigung in 10 ml Wasser und verdünnter Natronlauge bei pH 10 gelöst. Die Lösung wird mit 2N Salzsäure auf pH 1 eingestellt und über Nacht im Kühlschrank stehen gelassen, worauf sich ein farbloser Niederschlag bildet, der abfiltriert und getrocknet die 134 mg (0.33 mMol) der Titelverbindung ergibt; Smp. > 280°.

Das Ausgangsmaterial kann beispielsweise folgendermassen hergestellt werden:

### a) 5-Brom-2,3-diamino-toluol

Eine Lösung von 15 g (64.9 mmol) 4-Brom-2-methyl-6-nitro-anilin in 300 ml Ethanol wird in Anwesenheit von 1.5 g Raney-Nickel 4 Stunden bei etwa 27° hydriert. Anschliessend wird das Reaktionsgemisch filtriert und eingedampft. Die Titelverbindung wird als braunes Öl erhalten.
¹H-NMR (250 MHz, CDCl₃); δ = 6.76, 6.73 (2d, 2H), 3.22 (S, 2NH₂), 2.14 (S, Me).

### b) 7-Brom-5-methyl-1,2,3,4-tetrahydrochinoxalin-2,3-dion

13.05 g (64.9 mMol) 5-Brom-2,3-diamino-toluol und 6.42 g (1.1 Val) Oxalsäure werden bei Rückfluss 16 Stunden in 2N Salzsäure rühren gelassen. Das Gemisch wird abgekühlt, der Feststoff abfiltriert und mit Wasser gewaschen. Man erhält die Titelverbindung als braunen Feststoff.
¹H-NMR (250 MHz, DMSO); δ = 11.98, 11.32 (2s, 2NH), 7.13 (s, 2H), 2.33 (s, Me).

### c) 7-Brom-2,3-dichlor-5-methyl-chinoxalin

17 g (66.6 mmol) 7-Brom-5-methyl-1,2,3,4-tetralhydro-chinoxalin-2,3-dion werden in 80 ml Phosphoroxychlorid fünf Stunden bei Rückfluss und 40 Stunden bei 20° rühren gelassen. Das Gemisch wird eingedampft und unter Hochvakuum getrocknet. Der Rückstand wird vorsichtig mit einer gesättigter Kaliumcarbonatlösung versetzt, der Feststoff abfiltriert und mit Wasser gewaschen. Man erhält die Titelverbindung als braunen Feststoff.
¹H-NMR (250 MHz, DMSO); δ = 8.16, 7.99 (2d, 2H), 2.63 (s, Me).

### d) 7-Brom-5-methyl-2,3-dimethoxy-chinoxalin

2.97 g (129.5 mMol) Natrium werden in 100 ml Methanol gelöst. Diese Lösung wird zu 18.9 g (64.7 mmol) 7-Brom-5-methyl-2,3-dichlor-chinoxalin in 60 ml Methanol zugegeben und 20 Stunden zum Rückfluss erwärmt. Das Gemisch wird abgekühlt und mit 15 ml Wasser versetzt. Der Feststoff wird abfiltriert und mit Methanol und Wasser gewaschen. Man erhält die Titelverbindung als beigen Feststoff.
¹H-NMR (250 MHz, DMSO); δ = 7.73, 7.58 (2d, 2H), 4.05, 4.03 (2s, 2Me), 2.58 (s, Me).

### e) 7-Brom-5-brommethyl-2,3-dimethoxy-chinoxalin

15 g (53 mmol) 7-Brom-5-methyl-2,3-dimethoxy-chinoxalin, 9.9 g (1.05 Val) N-Bromsuccinimid und 0.87 g (0.1 Val) Azo-isobutyronitril werden in 100 ml Tetrachlorkohlenstoff gelöst und 24 Stunden bei Rückfluss rühren gelassen. Der Feststoff wird abfiltriert und das Filtrat mit Dichlormethan verdünnt. Es wird mit Wasser und Sole je einmal gewaschen. Die organische Phase wird über Magnesiumsulfat getrocknet und eingedampft. Der Rückstand wird mit Essigsäureethylester und Hexan umkristallisiert. Man erhält die Titelverbindung als leicht orange Kristalle.
¹H-NMR (250 MHz, CDCl₃); δ = 7.90, 7.68 (2d, 2H), 4.95 (s, 2H), 4.20, 4.13 (2s, 2Me).

### f) 5-Azidomethyl-7-brom-2,3-dimethoxy-chinoxalin

Zu 2.07 g (5.72 mmol) 7-Brom-5-brommethyl-2,3-dimethoxy-chinoxalin in 25 ml Dimethylformamid werden 743 mg (2 Val) Natriumazid bei 20° zugegeben. Nach 3 Stunden wird das Gemisch in Wasser gegossen, mit Diethylether extrahiert, mit Wasser und Sole gewaschen, und mit Magnesiumsulfat getrocknet. Das Lösungsmittel wird eingedampft.
¹H-NMR (250 MHz, CDCl₃); δ = 7.92, 7.58 (2d, 2H), 4.80 (s, 2H), 4.18, 4.13 (2s, 2Me).

### g) 5-Aminomethyl-7-bromo-2,3-dimethoxy-chinoxalin

4.47 g (13.8 mMol) 5-Azidomethyl-7-brom-2,3-dimethoxy-chinoxalin werden in 35 ml Tetrahydrofuran gelöst und 3.98 g (1.1 Val) Triphenylphosphin zugegeben. Das Gemisch wird bei 20° 4 Stunden rühren gelassen. 746 mg Wasser werden zugegeben und das Gemisch drei weitere Stunden gerührt. Der Feststoff wird abfiltriert und das Filtrat mit Essigsäureethylester und Natriumcarbonatlösung extrahiert. Die organischen Phasen werden vereinigt, mit Sole gewaschen, über Magnesiumsulfat getrocknet und eingedampft. Der Rückstand wird an Kieselgel mit Essigsäureethylester/Petrolether 1:1 chromatographiert.
¹H-NMR (250 MHz, CDCl₃); δ = 7.85, 7.53 (2d, 2H), 4.22 (s, 2H), 4.12 (s, 2Me).

### h) 1,3,5-Tri-N-(7-brom-2,3-dimethoxy-chinoxalin-5-ylmethyl)-[1,3,5]-triazinan

2.98 g (10 mMol) (7-brom-2,3-dimethoxy-chinoxalin-5-ylmethyl)-amin werden in 40 ml Ethanol durch Erwärmen gelöst. Nach dem Abkühlen auf Raumtemperatur wird der leicht gelben Lösung 1 ml Formalinlösung (37% in Wasser) zugetropft. Nach beendeter Zugabe fällt das Produkt in Form eines farblosen Niederschlages aus. Nach 3 Stunden Rühren wird der Niederschlag abfiltriert. Nach dem Trocknen am HV wird die Titelverbindung als farblose amorph kristalline Masse erhalten.
¹H-NMR (300 MHz, CDCl₃); δ = 7.83 (d, 2.3 Hz, 3H), 7.72 (d, 2.3 Hz, 3H), 4.24 (s, 6H), 4.13 (s, 9H), 4.04 (s, 9H), 3.69 (br. s, 6H). MS(FAB): 930, 932

### i) N-(7-Brom-2 3-dimethoxy-chinoxalin-5-ylmethyl)-aminomethylphosphonsäuredimethylester

Unter einer Stickstoffatmosphäre werden bei 0° 975 ml (10.64 mMol) Dimethylphosphit, 1.27 ml (9.19 mMol) Triethylamin und 1.47 ml (11.6 mMol) Trimethylsilylchlorid während 90 Minuten in 200 ml Chloroform gerührt. Innerhalb einer Stunde wird die Lösung bei 0° zu 3.0 g (3.22 mMol) in 200 ml Chloroform gelöstem 1,3,5-Tri-N-(7-brom-2,3-dimethoxy-chinoxalin-5-ylmethyl)-[1,3,5]-triazinan zugetropft. Nach 16 h Rühren bei Raumtemperatur wird die Suspension auf eiskalte Salzsäure (0.1 N in Wasser) gegossen und mit 3 Teilen Ether versetzt. Die organische Phase wird erschöpfend mit 0.1 N wässriger Salzsäure ausgeschüttelt. Die vereinigten wässrigen Phasen werden mit Kaliumcarbonatlösung auf pH 12-13 eingestellt und 6 mal mit Dichlormethan extrahiert. Nach dem Trocknen über Natriumsulfat und dem Einengen der organischen Phase werden 3.65 g leicht gelb gefärbter Kristalle der Titelverbindung erhalten.
¹H-NMR (300 MHz, CDCl₃); δ = 7.88 (d, 2.3 Hz, ¹H), 7.54 (d, 2.3 Hz, ¹H), 4.25 (s, 2H), 4.15 (s, 3H), 4.14 (s, 3H), 3.78 (d, 10 Hz, 6H), 2.95 (d. 13.1 Hz, 2H), MS(ES⁺) 422, 420 (MH⁺)

### j) N-Acetyl-N-(7-brom-2,3-dimethoxy-chinoxalin-5-ylmethyl)- aminomethylphosphonsäuredimethylester

Die auf 0° gekühlte Lösung von 420 mg (1 mMol) N-(7-brom-2,3-dimethoxy-chinoxalin-5-ylmethyl)-α-aminophosphonsäuredimethylester in 15 ml Tetrahydrofuran wird nacheinander mit 0.18 ml (1.3 mMol) Triethylamin und 0.13 ml (1.1 mMol) Acetylchlorid versetzt. Die farblose Suspension wird 16 h bei zunächst 0° und dann bei Raumtemperatur gerührt und schliesslich eingeengt. Der Rückstand wird in Dichlormethan aufgenommen und mit 0.1 N Salzsäure gewaschen. Die organische Phase wird über Natriumsulfat getrocknet, eingeengt und über eine Kieselgelsäule mit Essigester als Laufmittel gereinigt. Nach dem Einengen und Trocknen werden 400 mg (0.87 mMol) der Titelverbindung als farbloses Öl isoliert.
¹H-NMR (300 MHz, CDCl₃); δ = 7.91 (d, 2.2 Hz, 0.7H), 7.89 (d, 2.3 Hz, 0.3H), 7.59 (d, 2.3 Hz, 0.3H), 7.32 (d, 2.2 Hz, 0.7H), 5.22.(s, 0.6H), 5.20 (s, 1.4H), 4.16 (s, 0.9H), 4.14 (s, 4.2H), 4.13 (s, 0.9H), 3.92 (d, 11.2 Hz, 1.4H), 3.82 (d, 10.8 Hz, 1.8H), 3.79 (d, 10.9 Hz, 4.2H), 3.78 (d, 14.2 Hz, 0.6H), 2.27 (s, 0.6H), 2.20 (s, 1.4H). MS (ES⁺) 464, 462 (MH⁺)

### Beispiel 2: N-Acetyl-N-(7-chlor-2,3-dioxo-1,2,3,4-tetrahydrochinoxalin-5-ylmethyl)-aminomethylphosphonsäure

Die Titelverbindung kann in analoger Weise wie unter Beispiel 1 beschrieben hergestellt werden. Das dabei in Stufe 1f) einzusetzende Zwischenprodukt 5-Brommethyl-7-chlor-2,3-dimethoxy-chinoxalin kann beispielsweise folgendermassen hergestellt werden:

### a) 7-Chlor-5-methyl-1.4-dihydro-chinoxalin-2,3-dion

123 g (0.79 Mol) 2,3-Diamino-5-chlor-toluol und 106.2 g (1.18 Mol) Oxalsäure werden in 800 ml 4N Salzsäure während 5 Stunden am Rückfluss erhitzt. Man lässt das Reaktionsgemisch abkühlen, verdünnt mit Wasser, nutscht ab und wäscht mit Wasser nach. Dann wird das Produkt in heissem Ethanol verrührt, abgenutscht und das Nutschgut im Vakuum bei 60° getrocknet. Man erhält die Titelverbindung als leicht gräuliche Kristalle vom Smp. > 250°.

### b) 2,3,7-Trichlor-5-methyl-chinoxalin

155 g (0.74 Mol) 7-Chlor-5-methyl-1,4-dihydrochinoxalin-2,3-dion und 321.8g (1.55 Mol) Phosphorpentachlorid werden in 950 ml Phosphoroxychlorid während 36 Stunden am Rückfluss gerührt. Man destilliert das Phosphoroxychlorid ab und giesst den Rückstand auf 3 I Eiswasser. Die entstandene Suspension wird verrührt, abgenutscht und mit Wasser nachgwaschen. Nach der Trocknung des Nutschgutes im Vakuum bei 60° erhält man die Titelverbindung als bräunliche Kristalle, die ohne weitere Reinigung verwendet wird.

### c) 7-Chlor-2,3-dimethoxy-5-methyl-chinoxalin

30 g (0.121Mol) 2,3,7-Trichlor-5-methyl-chinoxalin werden in 330 ml Methanol unter Argon bei Raumtemperatur vorgelegt. Dazu tropft man 67.9 ml (0.364 Mol) einer ca. 5.4 molaren Lösung von Natrium-methoxid in Methanol und rührt das Gemisch 4.5 Stunden am Rückfluss. Nach Abkühlen auf 0° C nutscht man die Suspension ab, wäscht den Filterrückstand mit Methanol und trocknet im Vakuum bei 60° C. Man erhält die Titelverbindung als bräunliche Kristalle vom Smp. 94-96° C.

### d) 5-Brommethyl-7-chlor-2,3-dimethoxychinoxalin

10.0 g (41.9 mM) 7-Chlor-2,3-dimethoxy-5-methyl-chinoxalin werden in 160 ml Chlorbenzol unter Argon bei Raumtemperatur vorgelegt. Man gibt 8.6 g (48.2 mM) N-Bromsuccinimid und 0.69 g (4.2 mM) Azoisobutyronitril hinzu und rührt 18 Stunden bei 80° C. Nach Abkülhlen des Reaktionsgemisches auf Raumtemperatur wird das Chlorbenzol abdestilliert, der Rückstand mit Diäthyläther versetzt und die entstandene Suspension abgenutscht. Das Filtrat engt man ein, kristallisiert den Rückstand aus n-Hexan und erhält die Titelverbindung als farblose Kristalle vom Smp. 114-116°.

### Beispiel 3: N-Acetyl-N-(7-fluor-2,3-dioxo-1,2,3,4-tetrahydrochinoxalin-5- ylmethyl)-aminomethylphosphonsäure

Die Titelverbindung kann in analoger Weise wie unter Beispiel 1 beschrieben hergestellt werden. Das dabei in Stufe g) einzusetzende Zwischenprodukt 5-Aminomethyl-7-fluor-2,3-dimethoxy-chinoxalin kann beispielsweise folgendermassen hergestellt werden:

### a) 2,3-Diamino-5-fluor-toluol

25 g ( 0.147 Mol) 4-Fluor-2-methyl-6-nitro-anilin in 250 ml Tetrahydrofuran werden in Anwesenheit von 8 g Raney-Nickel bei ca. 34°C 2 Stunden hydriert. Anschliessend wird das Reaktionsgemisch abfiltriert und eingeengt. Man erhält die Titelverbindung als braunes Öl. ¹H-NMR (200 MHz, CDCl₃) 8 = 6.32-6.38 (2H), 3.25 (s, 2NH₂), 2.1 (s, Me).

### b) 7-Fluor-5-methyl-1,2,3,4-tetrahydrochinoxalin-2,3-dion

20 g (0.118 Mol) 2,3-Diamino-5-fluor-toluol und 15.8 g (0.176 Mol) Oxalsäure werden bei Rückfluss 16 Stunden in 4N Salzsäure gerührt. Das Reaktionsgemisch wird abgekühlt, mit Wasser verdünnt, abgenutscht und mit Wasser nachgewaschen. Man erhält die Titelverbindung als beige Kristalle vom Smp. >300° C.

### c) 2,3-Dichlor-7-fluor-5-methyl-chinoxalin

25 g (0.129 Mol) 7-Fluor-5-methyl-1,2,3,4-tetrahydrochinoxalin-2,3-dion werden in 170 ml Phosphoroxychlorid vorgelegt. Man gibt 56.3 g (0.27 Mol) Phosphorpentachlorid dazu und rührt 16 Stunden unter Rückfluss nach. Überschüssiges Phosphoroxychlorid wird aus dem Reaktionsgemisch abdestilliert.Der dunkelbraune Rückstand wird abgekühlt und auf 1000 ml Eiswasser gegossen. Die Suspension wird abgenutscht, mit viel Wasser nachgewaschen und das Nutschgut im Vakuum bei 60°C getrocknet. Man erhält die Titelverbindung als braune Kristalle vom Smp. 116-120°C.

### d) 7-Fluor-5-methyl-2,3-dimethoxy-chinoxalin

14 g (60.6 mmol) 2,3-Dichlor-7-fluor-5-methyl-chinoxalin werden in 165 ml Methanol vorgelegt. Tropfenweise wird eine ca. 5.4 M Lösung von Natriummethanolat in Methanol zugegeben. Man erwärmt auf Rückfluss und lässt 18 Stunden nachrühren. Das Reaktionsgemisch wird auf 0° C abgekühlt, die Suspension abgenutscht, mit kaltem

Methanol nachgewaschen und das Nutschgut im Vakuum bei 60° C getrocknet. Das Rohprodukt wird aus Hexan umkristallisiert. Man erhält die Titelverbindung als weisse Kristalle vom Smp. 107-109°C.

### e) 5-Brommethyl-2,3-dimethoxy-7-fluor-chinoxalin

8.4 g (37.8 mMol) 2,3-Dimethoxy-7-fluor-5-methyl-chinoxalin, 7.4 g (41.6 mmol) N-Bromsuccinimid und 0.63 g (0.38 mMol) Azo-isobutyronitril werden in 140 ml Tetrachlorkohlenstoff vorgelegt und 6 Stunden am Rückfluss nachgerührt. Das Reaktionsgemisch wird abgekühlt, eingeengt und der Rückstand in Diethylether aufgenommen. Man filtriert die Suspension ab und engt die Mutterlauge wieder ein. Das zurückgebliebene Rohprodukt wird aus Hexan umkristallisiert. Man erhält die Titelverbindung als weisse Kristalle vom Smp. 122-125°C.

### f) 5-Azidomethyl-2,3-dimethoxy-7-fluor-chinoxalin

Zu 4.0 g (13.3 mMol) 5-Brommethyl-2,3-dimethoxy-7-fluor-chinoxalin in 50 ml Dimethylformamid werden 1.73 g (26.6 mMol) Nariumazid bei Raumtemperatur zugegeben und 5h nachgerührt. Das Reaktionsgemisch wird auf Wasser gegossen, mit Diethylether extrahiert und mit Wasser und Sole gewaschen. Die organische Phase wird mit Na₂SO₄ getrocknet, abgenutscht und eingeengt. Man erhält die Titelverbindung als weisse Kristalle vom Smp. 75-78°C.

### g) 5-Aminomethyl-2,3-dimethoxy-7-fluor-chinoxalin

3.5 g (13.3 mMol) 5-Azidomethyt-2,3-dimethoxy-7-fluor-chinoxalin in 35 ml Tetrahydrofuran werden in Anwesenheit von 1.75 g Raney-Nickel ca. 19 Stunden bei Raumtemperatur hydriert. Das Reaktionsgemisch wird abfiltriert und eingeengt. Man erhält die Titelverbindung als gelbliche Kristalle.
¹H-NMR (300 MHz, DMSO); δ = 7.3-7.5 (2H), 4.15 (s, 2H), 4.02 (s, 6H).

### Beispiel 4: N-(2,3-Dioxo-7-nitro-1,2,3,4-tetrahydrochinoxalin-5-ylmethyl)-α-(ethylamino)-ethylphosphonsäure-hydrobromid

160 mg (0.37 mMol) N-(7-nitro-2,3-dimethoxy-chinoxalin-5-ylmethyl)-a-(ethylamino)-ethylphosphonsäuredimethylester werden in 8 ml Dichlormethan gelöst und mit 0.19 ml (4 Val) Trimethylbromsilan eine Stunde bei Raumtemperatur gerührt. Das Lösungsmittel und Überschuss Regenz werden eingedampft und der Rückstand kurz am Hochvakuum getrocknet, in 5 ml einer ca. 33%-ige Bromwasserstoff Lösung in Essigsäure gelöst, und 18 Stunden bei Raumtemperatur rühren gelassen. Das Reaktionsgemisch wird mit Diethylether verdünnt, der Feststoff abfiltriert, mit Diethylether gewaschen und getrocknet. Die Titelverbindung wird als beiger Feststoff erhalten; Smp. = 191 ° (Zers.).

Das Ausgangsmaterial kann beispielsweise folgendermassen hergestellt werden:

### a1) 5-Brommethyl-2,3-dimethoxy-chinoxalin

Die Titelverbindung kann in analoger Weise wie unter Beispiel 1c, 1d, 1e beschrieben, aus 5-Methyl-2,3-dioxo-1,2,3,4-tetrahydrochinoxalin hergestellt werden.

### b1) 5-Brommethyl-7-nitro-2.3-dimethoxy-chinoxalin

25 ml Schwefelsäure werden auf 0° abgekühlt und danach 9.5 g (33.55 mMol) 5-Brommethyl-2,3-dimethoxy-chinoxalin zugegeben. Nach weiterem 10 Minuten wird 3.39 ml (1 Val) Isopropylnitrat zugegeben und 1 Stunde bei 0° rühren gelassen. Das Gemisch wird auf Eis gegossen, der Feststoff abfiltriert und mit Wasser gewaschen. Man erhält die Titelverbindung als beigen Feststoff.
¹H-NMR (250 MHz, d₆-DMSO); δ = 8.62, 8.40 (2d, 2H), 5.02 (s, 2H), 4.27, 4.19 (2s, 2Me).

### c1) 5-[Di-(tert-bulyloxycarbonyl)amino]methyl-7-nitro-2,3-dimethomy-chinoxalin

10 g (30.5 mMol) 5-Brommethyl-7-nitro-2,3-dimethoxy-chinoxalin werden in 50 ml Dimethylformamid gelöst. 7.3 g (1.1 Val) Di-tert-butyl-iminodicarboxylat und 14.9 g (1.5 Val) Caesiumcarbonat werden zugefügt und das Reaktionsgemisch 10 Stunden auf 50° erhitzt, dann auf Raumtemperatur abgekühlt und mit Wasser und Essigester extrahiert. Die vereinigten organischen Phasen werden über Magnesiumsulfat getrocknet und eingeengt. Die Titelverbindung wird nach Säulenchromatographie mit Hexan/Essigester (9:1) als gelbes Öl erhalten.
¹H-NMR (CDCl₃, 250 MHz); δ = 8.55, 8.08 (2d, 2H); 5.34 (s, 2H), 4.18, 4.16 (2s, 2Me); 1.47 (s, 2 t-Bu).

### d1) 5-Aminomethyl-7-nitro-2,3-dimethoxy-chinoxalin

13.8g (29.7 mMol) 5-[Di-(*tert*-butyloxycarbonyl)amino]methyl-7-nitro-2,3-dimethoxy-chinoxalin wird in 60 ml Trifluoressigsäure 8 Stunden bei Raumtemperatur gerührt. Das Reaktionsgemisch wird unter vermindertem Druck eingeengt und das rote Öl bei 0° mit 1 N Kaliumcarbonat eine Stunde gut gerührt. Die gelben Kristalle werden abfiltriert, mit 100 ml Wasser und 100 ml eines 1:1 Gemisches von Essigester und Hexan gewaschen, und getrocknet.
¹H-NMR (CDCl₃, 250 MHz); δ = 8.57, 8.29 (2d, 2H); 4.33 (s, 2H); 4.20, 4.18 (2d, 2Me).

### e1) N-(7-nitro-2,3-dimethoxy-chinoxalin-5-ylmethyl)-α-aminoethylphosphonsäuredimethylester

500 mg (1.9 mMol) 5-Aminomethyl-7-nitro-2,3-dimethoxy-chinoxalin, 834 mg (3.7 Val) Magnesiumsulfat, 335 mg (1.3 Val) Kaliumcarbonat und 0.214 ml (2 Val) Acetaldehyd werden in 15 ml Dichlormethan 7 Stunden bei Raumtemperatur rühren gelassen. Das Reaktionsgemisch wird abfiltriert und das Filtrat eingedampft. Eine Lösung von 0.32 ml (1.2 Val) Triethylamin, 0.182 ml Dimethylphosphit und 0.36 ml (1.5 Val) Trmethylchlorsilan in 10 ml Dichlormethan wird bei 0° eine Stunde gerührt, der Rückstand in 10 ml Dichlormethan zugefügt und 14 Stunden reagieren gelassen. Zum Reaktionsgemisch wird Wasser zugegeben und die organische Phase abgetrennt. Die wässerige Phase wird zwei Mal mit Dichlormethan extrahiert und die vereinigten organischen Phasen über Magnesiumsulfat getrocknet. Die Titelverbindung wird nach Eindampfen des Lösungsmittel als gelbes Harz erhalten.
¹H-NMR (CDCl₃, 250 MHz); δ = 8.58, 8.34 (2d, 2H); 4.42, 4.34 (2d, 2H); 4.22, 4.19 (2d, 2Me); 3.82, 3.80 (2d, 2Me); 3.04 (m, ¹H); 1.38 (dd, Me).

### N-(7-nitro-2,3-dimethoxy-chinoxalin-5-ylmethyl)-α-aminoethylphosphonsäuredimethylester kann alternativ auch folgendermassen hergestellt werden:

### a2) 2,3-Dimethoxy-chinoxalin-5-carbaldehMd:

Zu einer Lösung von 3.7 g (163 mMol) Natrium in 700 ml Methanol werden 17 ml (188 mMol) 2-Nitropropan zugegeben. Nach 5-minütigem Rühren gibt man 35.5 g (125.4 mMol) festes 5-Brommethyl-2,3-dimethoxy-chinoxalin hinzu. Das Gemisch wird 1 Stunde auf Rückfluss erwärmt, wobei eine homogene Lösung entsteht. Nach Abkühlen wird die Lösung unter vermindertem Druck eingeengt. Der Rückstand wird in Essigester und 1 N Salzsäure aufgenommen, die Phasen werden getrennt und die organische Phase wird mit Wasser und Sole gewaschen, über Natriumsulfat getrocknet und eingeengt. Durch Kristallisation aus Essigester wird die Titelverbindung in Form weisser Kristalle isoliert. Smp. 137-140° ; DC (Essigsäureethylester/Hexan 1 : 3): Rf = 0.45.

### b2) 2,3-Dimethoxy-7-nitro-chinoxalin-5-carbaldehyd:

Zur einer auf 0° gekühlte Lösung von 22g (100.8 mMol) 5-Brommethyl-2,3-dimethoxy-chinoxalin in 88 ml Trifluoressigsäure wurden nachfolgend 44 ml 100-ige Salpetersäure, 44 ml 97%-ige Schwefelsäure und 44 ml Trifluoressigsäureanhydrid gegeben. Das Gemisch wurde 2 Stunden bei 0° gehalten und anschliessend vorsichtig auf ein Gemisch von 4N Natronlauge und Eis gegossen. Die Temperatur sollte nicht über 20° ansteigen. Das Gemisch wurde mit Essigester extrahiert, die organische Phase mit einer wässerigen 1 N Natronlauge, Wasser und Sole gewaschen und über Natriumsulfat getrocknet. Kristallisation des Rohprodukts ergab 18.8 g der Titelverbindung als leicht gelbe Kristalle. Smp.. = 147-149°; DC(SiO₂, EtOAc/Hexan 1 : 3): Rf = 0.25:

### c2) N-(7-nitro-2,3-dimethoxy-chinoxalin-5-ylmethyl)-α-aminoethylphosphonsäuredimethylester:

150 mg (0.569 mMol) 2,3-Dimethoxy-7-nitro-chinoxalin-5-carbaldehyd, 90 mg (1.04 Val) a-aminoethylphosphonsäuredimethylester und 500 mg (7.3 Val) Magnesiumsulfat werden in 5 ml DMSO gelöst und 24 Stunden bei Raumtemperatur gerührt. Das Reaktionsgemisch wird abfiltriert, das Filtrat eingedampft und der Rückstand in 5 ml Methanol gelöst. 0.027 ml (1 Val) Essigsäure, 78 mg (2 Val) Natriumacetat und 36 mg (1.2 Val) Natriumcyanoborhydrid werden zugegeben und bei Raumtemperatur 48 Stunden rühren gelassen. Das Reaktionsgemisch wird mit einer 1 N Salzsäure versetzt, und 30 Minuten rühren gelassen und dann mit Diethylether gewaschen. Die wässerige Phase wird mit einer 2N Natriumhydroxidlösung basisch gestellt und mit Essigester extrahiert. Die vereinigte Essigester-Phasen werden mit Sole und Magnesiumsulfat getrocknet und eingedampft. Die Titelverbindung wird als gelbes Harz erhalten.

### f) N-(7-nitro-2,3-dimethoxy-chinoxalin-5-ylmethyl)-α-(ethylamino)-ethylphosphonsäuredimethylester

300 mg (0.75 mMol) N-(7-Nitro-2,3-dimethoxy-chinoxalin-5-ylmethyl)-α-amino-ethylphosphonsäuredimethylester, 0.482 ml (8 Val) Ethyliodid und 1.4 ml (11 Val) Düsopropylethylamin werden in 10 ml Acetonitril 24 Stunden bei 70° rühren gelassen. Das Reaktionsgemisch wird eingedampft und der Rückstand in Diethylether verrührt. Der Feststoff wird abfiltriert, mit Diethylether gewaschen und das Filtrat eingedampft. Die Titelverbindung wird als bräunliches Öl erhalten.
¹H-NMR (CDCl₃, 250 MHz); δ = 8.63, 8.54 (2d, 2H); 4.37 (s, 2H); 4.19, 4.17 (2s, 2Me); 3.88, 3.73 (2d, 2Me); 3.27 (m,¹H); 2.9, 2.7 (2m, 2H); 1.39 (dd, Me); 1.13 (t, Me).

### Beispiel 5: N-Acetyl-N-(7-nitro-2,3-dioxo-1,2,3,4-tetrahydrochinoxalin-5-ylmethyl)-aminoethylphosphonsäure

Die Titelverbindung kann in analoger Weise wie in Beispiel 1 und 4 beschrieben, aber ausgehend von N-(7-nitro-2,3-dimethoxy-chinoxafin-5-ylmethyl)-aminoethylphosphonsäuredimethylester hergestellt werden; Smp. = 248°C.

### Beispiel 6: (R)-N-(7-Nitro-2,3-dioxo-1,2,3,4-tetrahydrochinoxalin-5-ylmethyl)-α-(ethylamino)-ethylphosphonsäure

4.24 g (9.9 mmol) (R)-N-(7-Nitro-2,3-dimethoxychinoxalin-5-ylmethyl)-α-ethylamino-ethylphosphonsäuredimethylester werden in 80 ml 6N Salzsäure 19 Stunden rühren gelassen. Das Reaktionsgemisch wird eingedampft und der Rückstand mit Wasser aufgeschlämmt. Die Titelverbindung wird als Feststoff vom Smp. = 218°C (Zers.) erhalten.

### Die Ausgangsmaterialien können beispielsweise wie folgt hergestellt werden:

### a) (R)-N-(7-Nitro-2,3-dimethoxychinoxelin-5-ylmethyl)-α-amino-ethylphosphonsäuredimethylester

5.14 g (19.5 mMol) 7-Nitro-2,3-dimethoxy-chinoxalin-5-carbaldehyd, 3.59 g (1.2 Val) L-Phosphoalanindimethylester und 18.8 g (8 Val) Magnesiumsulfat werden in 80 ml Dimethylsulfoxid 3 Stunden bei Raumtemperatur gerührt. Das Reaktionsgemisch wird abfittriert und eingedampft. Der Rückstand wird in 100 ml Methanol gelöst, und mit 1.12 ml (1 Val) Essigsäure, 3.2 g (2 Val) Natriumacetat und 1.71 g (1.4 Val) Natriumcyanoborohydrid versetzt. Das Reaktionsgemisch wird 18 Stunden bei Raumtemperatur gerührt, mit 1 N Salzsäure versetzt und mit Diethylether extrahiert. Die wässerige Phase wird mit 4N Natronlauge basisch gestellt und mit Essigester extrahiert. Die vereinigten organischen Phasen werden über Magnesiumsulfat getrocknet und eingedampft. Die Titelverbindung wird als braunes Öl erhalten;
MS (ES+): 801 (2M+1 ), 401 (M+1), 291.

### b) (R)-N-(7-Nitro-2,3-dimethoxychinoxalin-5-ylmethyl)-α-ethylamino-ethylphosphonsäuredimethylester

3.4 g (8.5 mMol) (R)-N-(7-Nitro-2,3-dimethoxychinoxalin-5-ylmethyl)-α-amino-ethylphosphonsäure-dimethylester, 8.9 ml (7.5 Val) Ethyliodid und 20.9 ml (14.5 Val) Hünigs Base werden in 18 ml Acetonitril gemischt und 18 Stunden bei 55°C rühren gelassen. Das Reaktionsgemisch wird eingedampft und mit Essigester aufgeschtemmt. Der ausgefallene Feststoff wird abfiltriert und mit Essigester gewaschen. Die Titelverbindung wird nach Eindampfen des Filtrats und Säulenchromatographie mit Essigester/Methanol (95:5) als Laufmittel in Form eines gelben Harzes erhalten.
NMR (250 MHz, CDCl₃): δ (ppm) = 8.60, 8.52 (2m, 2H); 4.33 (br.s, 2H); 4.17, 4.16 (2s, 2Me); 3.83, 3.73 (2d, 2MeO); 3.23 (dq, 1 H); 2.88, 2.70 (2m, 2H); 1.38 (dd, Me); 1.11 (t, Me).

### Beispiel 7: (S)-N-(7-Nitro-2.3-dioxo-1,2,3,4-tetrahydrochinoxalin-5-ylmethyl)-α-(ethylamino)-ethylphosphonsäure

Die Titelverbindung kann in analoger Weise wie in Beispiel 6 beschrieben hergestellt werden; Smp. = 219°C ((Zers.).).

### Beispiel 8: (R)-N-(7-Nitro-2,3-dioxo-1,2,3,4-tetrahydrochinoxalin-5-ylmethyl)-α-aminoethylphosphonsäure-hydrochlorid

Die Titelverbindung kann in analoger Weise wie in Beispiel 6, aber ohne Stufe b) beschrieben hergestellt werden; Smp. = 218°C ((Zers.).).

### Beispiel 9: (S)-N-(7-Nitro-2,3-dioxo-1,2,3,4-tetrahydrochinoxalin-5-ylmethyl)-α-aminoethylphosphonsäure-hydrochlorid

Die Titelverbindung kan in analoger Weise wie in Beispiel 6, aber ohne Stufe b) beschrieben hergestellt werden; Smp. = 218°C ((Zers.).).

### Beispiel 10: (R)-N-(7-Brom-2,3-dioxo-1,2,3,4-tetrahydrochinoxalin-5-ylmethyl)-α-aminoethylphosphonsäure

Die Titelverbindung kann in analoger Weise wie in Beispiel 6, aber ohne Stufe b) undausgehend von 5-Brommethyl-7-brom-2,3-dimethoxy-chinoxalin, hergestellt werden; Smp. = 272°C (Zers.).

### Beispiel 11: (S)-N-(7-Brom-2,3-dioxo-1,2,3,4-tetrahydrochinoxalin-5-ylmethyl)-α-aminoethylohosphonsäure

Die Titelverbindung kann in analoger Weise wie in Beispiel 6, aber ohne Stufe b) undausgehend von 5-Brommethyl-7-brom-2,3-dimethoxy-chinoxalin, hergestellt werden; Smp. = 278°C (Zers.)°C.

### Beispiel 12: In analoger Weise wie unter den Beispielen 3 und 4 beschrieben kann man ferner herstellen:

N-(7-Chlor-2,3-dioxo-1,2,3,4-tetrahydrochinoxalin-5-ylmethyl)-α-(methylamino)-ethylphosphonsäure-hydrobromid;
N-(7-Fluor-2,3-dioxo-1,2,3,4-tetrahydrochinoxalin-5-ylmethyl)-α-(ethylamino)-ethylphosphonsäure-hydrobromid;
N-(2,3-Dioxo-7-nitro-1,2,3,4-tetrahydrochinoxalin-5-ylmethyl)-α-(methylamino)-ethylphosphonsäure-hydrobromid, Smp. = 191 ° ((Zers.).);
N-(2,3-Dioxo-7-nitro-1,2,3,4-tetrahydrochinoxalin-5-yimethyl)- aminomethylphosphonsäure , Smp. = 272 °C (Zers.);
N-(2,3-Dioxo-7-nitro-1,2,3,4-tetrahydrochinoxalin-5-ylmethyl)-α-(ethytamino)-methylphosphonsäure-hydrobromid, Smp. = 280 - 285 °C (Zers.);
N-(2,3-Dioxo-7-nitro-1,2,3,4-tetrahydrochinoxalin-5-ylmethyl)-a-(methylamino)-methylphosphonsäure, Smp. > 286 °C (Zers.).

### Beispiel 13: N-(2,3-Dioxo-7-nitro-1,2,3,4-tetrahydrochinoxalin-5-ylmethyl)-β-amino-propylphosphonsäure-hydrobromid

Die Titelverbindung kann aus N-(2,3-Dimethoxy-7-nitro-chinoxalin-5-ylmethyl)-β-aminopropylphosphonsäuredimethylester in analoger Weise wie in Beispiel 2 hergestellt und, dann aus Dimethylformamid unter Zusatz von Ethanol und Diethylether umkristallisiert werden; Smp. = 282 (Zers.).

### Das Ausgangsmaterial kann beispielsweise folgendermaßen hergestellt werden:

### N-(2,3-Dimethoxy-7-nitro-chinoxalin-5-ylmethyl)-β-amino-propylphosohonsäuredimethylester

200 mg (0.757 mMol) 5-Aminomethyl-7-nitro-2,3-dimethoxy-chinoxalin, 547 mg (4.5 Val) Magnesiumsulfat und 163 mg (1.3 Val) Dimethyl-2-oxopropylphosphonat werden in 8 ml Dichlormethan 20 Stunden bei Raumtemperatur gerührt. Dann werden 4 ml Methanol, 0.095 ml Essigsäure und 52 mg (1.1 Val) Natriumcyanoborhydrid zugegeben und 4 Stunden rühren gelassen. Anschliessend wird das Reaktionsgemisch filtriert und das Filtrat mit Wasser und Sole extrahiert. Die organischen Phasen werden vereinigt, über Magnesiumsulfat getrocknet und eingedampft. Die Titelverbindung wird als braunes Öl erhalten.

### Beispiel 14: N-(2,3-Dioxo-7-nitro-1,2,3,4-tetrahydrochinoxalin-5-ylmethyl)-2-amino-phenylphosphonsäure-hydrobromid

Die Titelverbindung kann aus N-(2,3-Dimethoxy-7-nitro-chinoxalin-5-ylmethyl)-2-aminophenylphosphonsäurediethylester in analoger Weise wie in Beispiel 4 beschrieben erhalten werden; Smp. = 191 ° (Zers.).

Das Ausgangsmaterial kann beispielsweise folgendermaßen hergestellt werden:

### N-(2,3-Dimethoxy-7-nitro-chinoxalin-5-ylmethyl)-2-amino-phenylphosphonsäurediethylester:

190 mg (0.579 mMol) 5-Brommethyl-7-nitro-2,3-dimethoxy-chinoxalin, 159 mg (1.2 Val) 2-Amino-phenylphosphonsäurediethylester und 0.2 ml (2 Val) Düsopropyiethylamin werden in 8 ml Acetonitril 20 Stunden bei Rückfluss rühren gelassen. Das Reaktionsgemisch wird eingedampft und der Rückstand mit Wasser und Essigester extrahiert. Die vereinigten organischen Phasen werden mit Sole und Magnesiumsulfat getrocknet und eingedampft. Die Titelverbindung wird als gelber Feststoff erhalten.
¹H-NMR (CDCl₃, 250 MHz); δ = 8.57, 8.29 (2d, 2H); 7.48, 7.25, 6.67, 6.51 (4m, 4H); 4.93 (s, 2H), 4.24, 4.28 (2s, 2Me); 4.10 (m, 2CH₂); 1.32 (m, 2CH₃).

### Beisoiel 15: In analoger Weise wie unter Beispiel 1 bis 4, 13 und 14 beschrieben kann man femer herstellen:

N-(7-Brom-2,3-dioxo-1,2,3,4-tetrahydrochinoxalin-5-ylmethyl)-α-aminobenzylphosphonsäure, Smp. > 310°;
N-(7-Bromo-2,3-dioxo-1,2,3,4-tetrahydrochinoxalin-5-ylmethyl)-α-amino-3-methylbutylphosphonsäure-hydrobromid, Smp. = 254-256°;N-(7-Bromo-2,3-dioxo-1,2,3,4-tetrahydrochinoxalin-5-ylmethyl)-α-amino-isobutylphosphonsäure, Smp. = 249-251 °;
N-(7-Bromo-2,3-dioxo-1,2,3,4-tetrahydrochinoxalin-5-ylmethyl)-α-amino-β-benzyloxyethylphosphonsäure, Smp. > 280°C, MS (ES-): 484, 482 (M-1);
N-(7-Bromo-2,3-dioxo-1,2,3,4-tetrahydrochinoxalin-5-ylmethyl)-α-amino-propylphosphonsäure, Smp. = 264-266°;
N-(2,3-Dioxo-7-nitro-1,2,3,4-tetrahydrochinoxalin-5-ylmethyl)-4-amino-benzylphosphonsäurediethylester-hydrochlorid, MS(ES⁻): 461 (M-H)⁻:
N-(2,3-Dioxo-7-nitro-1,2,3,4-tetrahydrochinoxalin-5-ylmethyl)-α-amino-(3-hydroxybenzyl)-phosphonsäure-hydrobromid, Smp. > 280 °;
N-(2,3-Dioxo-7-nitro-1,2,3,4-tetrahydrochinoxalin-5-ylmethyl)-α-amino-(isopropyl)-phosphonsäure-hydrobromid, Smp. = 212° (Zers.);
N-(2,3-Dioxo-7-nitro-1,2,3,4-tetrahydrochinoxalin-5-ylmethyl)-4-amino-benzyl- . phosphonsäure-hydrobromid, DC (t-ButylOMe, MeOH, AcOH (80:18:2)): R₁ = 0.27;
*trans*-2-[N-(2,3-Dioxo-7-nitro-1,2,3,4-tetrahydrochinoxalin-5-ylmethyl)-amino]-cyclopropylphosphonsäure;
N-(2,3-Dioxo-7-nitro-1,2,3,4-tetrahydrochinoxalin-5-ylmethyl)-α-methylamino-(isopropyl)-phosphonsäure-hydrobromid, Smp. = 212° (Zers.);
*trans*-2-[N-(2,3-Dioxo-7-nitro-1,2,3,4-tetrahydrochinoxalin-5-ylmethyl)-aminoJ-cyclopropylphosphonsäure-hydrobromid, Smp. > 320° (Zers.);
N-(2,3-Dioxo-7-nitro-1,2,3,4-tetrahydrochinoxalin-5-ylmethyl)-α-amino-(3,4,5-trimethoxybenzyl)-phosphonsäure-hydrobromid, Smp. = 265°
N-(2,3-Dioxo-7-nitro-1,2,3,4-tetrahydrochinoxalin-5-ylmethyl)-α-amino-cyclohexylmethylphosphonsäure-hydrobromid, Smp. = 255° (Zers.);
N-(2,3-Dioxo-7-nitro-1,2,3,4-tetrahydrochinoxalin-5-ylmethyl)-α-amino-n-butylphosphonsäure-hydrobromid, Smp. = 230° (Zers.);
N-(2,3-Dioxo-7 -nitro-1 ,2,3,4-tetrahydrochinoxalin-5-ylmethyl)-α-amino-3-methylbutylphosphonsäure-hydrobromid, Smp. = 220° (Zers.);
N-(2,3-Dioxo-7-nitro-1,2,3,4-tetrahydrochinoxalin-5-ylmethyl)-α-amino-benzylphosphonsäure-hydrobromid, Smp. = 205° (Zers.);
N-(2,3-Dioxo-7-nitro-1,2,3,4-tetrahydrochinoxalin-5-ylmethyl)-α-amino-(3-thienyl)-methylphosphonsäure-hydrobromid, Smp. = 205° (Zers.);
N-(2,3-Dioxo-7-nitro-1,2,3,4-tetrahydrochinoxalin-5-ylmethyl)-a-amino-(4-methoxycarbonylbenzyl)-phosphonsäure-hydrobromid, Smp. = 270°;
N-(2,3-Dioxo-7-nitro-1,2,3,4-tetrahydrochinoxalin-5-ylmethyl)-α-amino-(4-carboxy-benzyl)-phosphonsäure-hydrobromid, Smp. = > 280 °C;
N-(2,3-Dioxo-7-nitro-1,2,3,4-tetrahydrochinoxalin-5-ylmethyl)-α-amino-(3-nitrobenzyl)phosphonsäure-hydrobromid, Smp. = 205 ° (Zers.);
N-(2,3-Dioxo-7-nitro-1,2,3,4-tetrahydrochinoxalin-5-ylmethyl)-α-amino-(2-hydroxy-3-methoxybenzyl)-phosphonsäure-hydrobromid, Smp. >330°;
N-(7-Chlor-2,3-dioxo-1,2,3,4-tetrahydrochinoxalin-5-ylmethyl)-2-aminophenylphosphonsäure-hydrobromid, Smp. > 250°;
N-(2,3-Dioxo-7-nitro-1,2,3,4-tetrahydrochinoxalin-5-ylmethyl)-α-amino-2-pyrrolylmethylphosphonsäure-hydrobromid, Smp. > 320;
N-(2,3-Dioxo-7-nitro-1,2,3,4-tetrahydrochinoxalin-5-ylmethyl)-α-amino-(3-methyl-sulfanylpropyl)-phosphonsäure-hydrobromid, Smp. 252°C (Zers.);
N-(2,3-Dioxo-7-nitro-1,2,3,4-tetrahydrochinoxalin-5-ylmethyl)-α-amino-(2-hydroxy-2-methylpropyl)-phosphonsäure-hydrobromid, Smp. > 256 °C;
N-(7-Nitro-2,3-dioxo-1,2,3,4-tetrahydrochinoxalin-5-ylmethyl)-2-aminophenylphosphonsäure-hydrobromid;
N-(7-Chlor-2,3-dioxo-1,2,3,4-tetrahydrochinoxalin-5-ylmethyl)-α-amino-ethylphosphonsäure Smp. > 270°C, MS (ES-): 332 (M-1), 250, 207;
N-(7-Fluor-2,3-dioxo-1,2,3,4-tetrahydrochinoxalin-5-ylmethyl)-α-amino-ethylphosphonsäure Smp. > 250°C, [¹H]-NMR (D₂O, 250 Mhz): δ(ppm) = 7.1 (m, 2H); 4.57 (m, 2H); 3.42 (m, 1 H); 1.47 (m, 3H).

### Beispiel 16: P-Benzyl N-(2,3-dioxo-7-nitro-1,2,3,4-tetrahydrochinoxalin-5-ylmethyl)-aminomethylphosphinsäure-hydrobromid

Die Titelverbindung wird wie in Beispiel 1 beschrieben aus P-Benzyl-N-(2,3-dimethoxy-7-nitro-chinoxalin-5-ylmethyl)-aminomethylphosphinsäurediethylester hergestellt; Smp. = 196° (Zers.).

Das Ausgangsmaterial kann beispielsweise wie folgt hergestellt werden:

### a) P-Benzyl-N-(2,3-dimethoxy-7-nitro-chinoxalin-5-ylmethyl)-aminomethilphosphinsäureethylester

300 mg (1.136 mMol) 5-Aminomethyl-7-nitro-2,3-dimethoxy-chinoxalin, 0.13 ml (2 Val) Acetaldehyd, 683 mg (5 Val) Magnesiumsulfat und 204 mg (1.3 Val) Kaliumcarbonat werden in 8 ml Dichlormethan zwei Stunden bei Raumtemperatur rühren gelassen. Das Reaktionsgemisch wird abfiltriert und zum Filtrat werden 0.205 ml (1.3 Val) Triethylamin, 0.215 ml (1.5 Val) Trimethylchlorsilan und 209 mg (1 Val) P-Benzyl phosphinsäureethylester zugegeben. Das Reaktionsgemisch wird 18 Stunden rühren gelassen und dann mit Wasser und Dichlormethan extrahiert. Die organische Phasen werden vereinigt, über Magnesiumsulfat getrocknet und eingedampft. Man erhält die Titelverbindung als gelbes Harz; MS(ES+): 353, 537 (M+1)⁺.

### Beispiel 17: In analoger Weise wie unter den Beispielen 1 und 16 beschrieben kann man ferner herstellen:

P-Methyl-N-(2,3-dioxo-7-nitro-1,2,3,4-tetrahydrochinoxalin-5-ylmethyl)-α-aminoethylphosphinsäure, Smp.. 226° (Zers.);
P-Benzyl-(7-nitro-2,3-dioxo-1,2,3,4-tetrahydrochinoxalin-5-yl)-aminomethanphosphinsäure;
P-Methyl-(7-nitro-2,3-dioxo-1,2,3,4-tetrahydrochinoxalin-5-yl)-aminomethanphosphinsäure.

### Beispiel 18: N-(7-Nitro-2,3-dioxo-1,2,3,4-tetrahydrochinoxalin-5-ylmethyl)-4-phosphonobuttersäureamid

Die Titelverbindung kann wie unter Beispiel 4, aber aus N-(2,3-Dimethoxy-7-nitro-chinoxalin-5-ylmethyl)-4-(dimethylphosphono)-buttersäureamid hergestellt werden; Smp. = 220:240 ° (Zers.).

Das Ausgangsmaterial kann beispielsweise wie folgt hergestellt werden:

### a) N-(2,3-Dimethoxy-7-nitro-chinoxalin-5-ylmethyl)-4-(dimethylphosphono)-buttersäureamid

Zur Lösung von 150 mg (0.568 mMol) 5-Aminomethyl-2,3-dimethoxy-7-nitro-chinoxalin und 165 mg (0.738 mMol) 4-(Dimethylphosphono)-buttersäure in 3 ml Methylenchlorid werden 163 mg (0.851 mMol) N-(Dimethylaminopropyl)-N'-ethyl-carbodiimid-hydrochlorid bei Raumtemperatur gegeben und das Gemisch 30 Stunden gerührt. Danach wird das Gemisch mit Methylenchlorid verdünnt und mit 0.2 N Salzsäure und Sole gewaschen, am Rotationsverdampfer eingeengt und der Rückstand am Hochvakuum getrocknet. Man erhält die Titelverbindung als gelbes Harz.

### Beispiel 19: In analoger Weise wie unter Beispiel 18 beschrieben kann man ferner herstellen:

N-(7-Nitro-2,3-dioxo-1,2,3,4-tetrahydrochinoxalin-5-ylmethyl)-2-phosphono-essigsäureamid, Smp. = 280:283 ° (Zers.);
N-(7-Nitro-2,3-dioxo-1,2,3,4-tetrahydrochinoxalin-5-ylmethyl)-3-phosphonopropensäureamid, Smp. 240-260° (Zers.);
N-(7-Nitro-2,3-dioxo-1,2,3,4-tetrahydrochinoxalin-5-ylmethyl)-2-phosphono-indan-2-carbonsäureamid, Smp. 280-290° (Zers.) ;

### Beispiel 20: N-Benzyl-N-(2,3-dioxo-7-nitro-1,2,3,4-tetrahydrochinoxalin-5-ylmethyl)-aminomethylphosphonsäure-hydrobromid

0.7 g (1.4 mMol) N-Benzyl-N-(2,3-dimethoxy-7-nitro-chinoxalin-5-ylmethyl)-α-amino-methylphosphonsäurediethylester und 1.1 ml (8.3 mmol) Trimethylsilylbromid werden in 7 ml Methylenchlorid 16 Stunden bei Raumtemperatur gerührt. Anschliessend fügt man 7 ml Ethanol dazu und rührt weitere 24 Stunden bei Raumtemperatur. Das Reaktionsgemisch wird eingedampft, der Rückstand in 7 ml Essigsäure gelöst, 7 ml 33%-ige HBr in Essigsäure zugegeben und das Gemisch 4 Stunden bei Raumtemperatur gerührt. Nach Zugabe von 60 ml Aether bildet sich eine Suspension, die abgenutscht wird. Das Nutschgut wäscht man mit Aether nach und trocknet bei 60° im Vakuum. Anschliessend werden die Kristalle in Essigsäureäthylester verrührt und wiederum abfiltriert. Man erhält die Titelverbindung als gräuliche Kristalle vom Smp. 238-240° (Zersetzung).

Das Ausgangsmaterial kann beispielsweise wie folgt hergestellt werden:

### a) N-Benzyl-N-(2,3-dimethoxy-7-nitro-chinoxalin-5-ylmethyl)-amino-methylphosphonsäurediethylester

1.0 g (3.05 mmol) 5-Brommethyl-2,3-dimethoxy-7-nitro-chinoxalin, 1.1 g (3.66 mMol) Benzylamino-methanphosphonsäure-diäthylester-hydrochlorid und 1.9 ml (10.98 mmol) N-Ethyldiisopropylamin werden in 10 ml Dimethylformamid unter Argon ca. 18 Stunden bei Raumtemperatur und noch 3 Stunden bei 80° C verrührt. Nach Zugabe von Essigsäureäthylester wird mit Wasser und Sole extrahiert, die Wasserphasen mit Essigsäureäthylester gewaschen, die organischen Phasen vereinigt, mit Natriumsulfat getrocknet, abgenutscht und eingeengt. Der Eindampfrückstand wird an Kieselgel mit Hexan-Essigsäureäthylester-(1:1) chromatographiert. Man erhält 0.71 g (46 %) N-Benzyl-N-(2,3-dimethoxy-7-nitro-chinoxalin-5-ylmethyl)-aminomethylphosphonsäurediethylester.

### Beispiel 21: In analoger Weise wie in Beispiel 20 beschrieben werden auch die folgenden Verbindungen hergestellt:

N-Benzyl-N-(7-brom-2,3-dioxo-1,2,3,4-tetrahydrochinoxalin-5-ylmethyl)-amino-methylphosphonsäure;
N-Benzyl-N-(7-chlor-2,3-dioxo-1,2,3,4-tetrahydrochinoxalin-5-ylmethyl)-amino-methylphosphonsäure, Smp. 245-248° (Zers.);
N-Benzyl-N-(2,3-dioxo-7-fluor-1,2,3,4-tetrahydrochinoxalin-5-ylmethyl)-amino-methylphosphonsäure.

### Beispiel 22: N-Benzyl-N-(2,3-dioxo-7-nitro-1,2,3,4-tetrahydrochinoxalin-5-ylmethyl)-α-aminoethanphosphonsäure

Die Titelverbindung, Smp. 254-257 (Zers.), kann ausgehend von 5-Brommethyl-2,3-dimethoxy-7-nitro-chinoxalin und 2-Benzylamino-ethanphosphonsäurediethylesterin analoger Weise wie Beispiel 1 beschrieben erhalten werden.

### Beispiel 23: N-(2,3-Dioxo-7-fluor-1,2,3,4-tetrahydrochinoxalin-5-ylmethyl)-α-aminoethylphosphonsäure

Die Titelverbindung kann durch Solvolyse von N-(2,3-dimethoxy-7 -fluor-chinoxalin-5-ylmethl)-α-aminoethylphosphonsäuredimethylester in analoger Weise wie in Beispiel 1 beschrieben erhalten werden. Das Ausgangsmaterial (N-(2,3-dimethoxy-7-fluor-chinoxalin-5-ylmethl)-α-aminoethylphosphonsäuredimethylester) kann in analoger Weise wie in Beispiel 4, e1) hergestellt werden:
¹H-NMR (CDCl₃, 300 MHz); δ = 7.2-7.4 (2H), 4.2-4.4 (2H), 4.12 (s, 6H), 3.75 (6H), 3.0 (m, 1 H), 1.28-1.45 (m, 3H).

### Beispiel 24: In analoger Weise wie in den Beispielen 1 bis 23 beschrieben werden auch die folgenden Verbindungen hergestellt:

(R)-N-(7-Nitro-2,3-dioxo-1,2,3,4-tetrahydrochinoxalin-5-ylmethyl)-β-amino-propylphosphonsäure-hydrochlorid, Smp. = 293°C (Zers);
(S)-N-(7-Nitro-2,3-dioxo-1,2,3,4-tetrahydrochinoxalin-5-ylmethyl)-β-amino-propylphosphonsäure-hydrochlorid, Smp. = 295°C (Zers);N-(2,3-Dioxo-7-nitro-1,2,3,4-tetrahydro-chinoxalin-5-ylmethyl)-o amino-propylphosphonsäure-hydrochlorid, Smp. = 235°C (Zers.);
N-(2,3-Dioxo-7-nitro-1,2,3,4-tetrahydro-chinoxalin-5-ylmethyl)-α-amino-(tetrahydropyran-4-yl)-phosphonsäure-hydrochlorid, Smp. = 310°C (Zers.);
N-(2,3-Dioxo-7-nitro-1,2,3,4-tetrahydro-chinoxalin-5-ylmethyl)-α-amino-(piperidin-4-yl)-phosphonsäure-dihydrochlorid, Smp. =251°C (Zers.);
N-(2,3-Dioxo-7-nitro-1,2,3,4-tetrahydro-chinoxalin-5-ylmethyl)-α-amino-(furan-2-ylmethyl)-phosphonsäure-hydrochlorid;
N-(2,3-Dioxo-7-nitro-1,2,3,4-tetrahydro-chinoxalin-5-ylmethyl)-α-amino-(2-methoxy)-ethylphosphonsäure-hydrochlorid;
N-(2,3-Dioxo-7-nitro-1,2,3,4-tetrahydro-chinoxalin-5-ylmethyl)-2-amino-2-cyclohexyl-ethylphosphonsäure-hydrochlorid;
N-(2,3-Dioxo-7-nitro-1,2,3,4-tetrahydro-chinoxalin-5-ylmethyl)-β-amino-isopropyl-phosphonsäure-hydrochlorid;
N-(2,3-Dioxo-7-nitro-1,2,3,4-tetrahydro-chinoxalin-5-ylmethyl)-2-amino-cyclohexyl-phosphonsäure-hydrochlorid;
N-(2,3-Dioxo-7-nitro-1,2,3,4-tetrahydro-chinoxalin-5-ylmethyl)-2-amino-2-methyl-propylphosphonsäure-hydrochlorid;
N-(2,3-Dioxo-7-nitro-1,2,3,4-tetrahydro-chinoxalin-5-ylmethyl)-2-am ino-butyl-phosphonsäu rehydrochlorid;
N-(2,3-Dioxo-7-nitro-1,2,3,4-tetrahydro-chinoxalin-5-ylmethyl)-2-amino-heptyl-phosphonsäure-hydrochlorid, MS(FB+): 415 (M+1) ;
N-(2,3-Dioxo-7-nitro-1,2,3,4-tetrahydro-chinoxalin-5-ylmethyl)-2-amino-3-phenyloxy-propylphosphonsäure-hydrochlorid, Smp. = 234°C (Zers.) ;
N-(2,3-Dioxo-7-nitro-1,2,3,4-tetrahydro-chinoxalin-5-ylmethyl)-2-ethylamino-ethylphosphonsäure, Smp. = 286°C (Zers.) ;
N-(2,3-Dioxo-7-nitro-1,2,3,4-tetrahydro-chinoxalin-5-ylmethyl)-2-benzylamino-ethylphosphonsäure Smp. = 225°C (Zers.) ;
N-(2,3-Dioxo-7-nitro-1,2,3,4-tetrahydro-chinoxalin-5-ylmethyl)-2-amino-2-*p*-tolyl-ethylphosphonsäure-hydrochlorid;
N-(2,3-Dioxo-7-nitro-1,2,3,4-tetrahydro-chinoxalin-5-ytmethyi)-2-amino-2-(2-methoxyphenyl)-ethylphosphonsäure-hydrochlorid;
N-(2,3-Dioxo-7-nitro-1,2,3,4-tetrahydro-chinoxalin-5-yfmethyl)-2-amino-2-(4-fluorphenyl)-ethylphosphonsäure-hydrochlorid;
N-(2,3-Dioxo-7-nitro-1,2,3,4-tetrahydro-chinoxalin-5-ylmethyl)-2-amino-2-phenyl-ethylphosphonsäure-hydrochlorid, Smp. = 258°C (Zers.) ;
P-Methyl-N-(2,3-dioxo-7-nitro-1,2,3,4-tetrahydro-chinoxalin-5-ylmethyl)-2-amino-2-phenyl-ethylphosphinsäure-hydrochlorid, Smp. = 258°C (Zers.) ;
N-(2,3-Dioxo-7-nitro-1,2,3,4-tetrahydro-chinoxalin-5-ylmethyl)-2-amino-1-phenyl-ethylphosphonsäure-hydrochlorid, Smp. = 262°C (Zers.) ;
N-(2,3-Dioxo-7-nitro-1,2,3,4-tetrahydro-chinoxalin-5-ylmethyl)-2-amino-1-(furan-2-yl)-ethylphosphonsäure-hydrochlorid;
N-(2,3-Dioxo-7-nitro-1,2,3,4-tetrahydro-chinoxalin-5-yl methyl)-2-amino-1-(4-fluor-phenyl)-ethylphosphonsäure-hydrochlorid;
N-(2,3-Dioxo-7-nitro-1,2,3,4-tetrahydro-chinoxalin-5-ylmethyl)-2-amino-1-(4-methoxy-phenyl)-ethylphosphonsäure-hydrochlorid;
N-(2,3-Dioxo-7-nitro-1,2,3,4-tetrahydro-chinoxalin-5-ylmethyl)-2-amino-1-(3-methoxy-phenyl)-ethylphosphonsäure-hydrochlorid;
N-(2,3-Dioxo-7-nitro-1,2,3,4-tetrahydro-chinoxalin-5-ylmethyl)-2-amino-1 -(2-chlor-phenyl)-ethylphosphonsäure-hydrochlorid;
N-(2,3-Dioxo-7-nitro-1,2,3,4-tetrahydro-chinoxalin-5-ylmethyl)-2-amino-1-(4-tolyl)-ethylphosphonsäure-hydrochlorid;
N-Benzyl-N-(7-brom-2,3-dioxo-1,2,3,4-tetrahydrochinoxalin-5-ylmethyl)-α-amino-ethylphosphonsäure;
{1-[(7-Bromo-2,3-dioxo-1,2,3,4-tetrahydro-quinoxalin-5-ylmethyl)-amino]-cyclopropyl}-phosphonsäure, Smp. = 295°C (Zers);
N-Benzyl-N-(7-chlor-2,3-dioxo-1,2,3,4-tetrahydrochinoxalin-5-ylmethyl)-α-amino-ethylphosphonsäure;
N-Benzyl-N-(2,3-dioxo-7-fluor-1,2,3,4-tetrahydrochinoxalin-5-ylmethyl)-α-amino-ethylphosphonsäure.

### Beispiel 25: In analoger Weise wie in den Beispielen 1 bis 23 beschrieben können ausgehend von 7-Cyano-5-methyl-2,3-dimethoxy-chinoxalin auch die folgenden Verbindungen hergestellt werden:

N-(7-Cyano-2,3-dioxo-1,2,3,4-tetrahydrochinoxalin-5-ylmethyl)-α-amino-ethylphosphonsäure, Smp. > 270°C, H-NMR (DMSO, 250 MHz): δ (ppm) = 7.62, 7.47 (2m, 2H);
4.50, 4.40 (2d, 2H); 3.22 (m, 1H); 1.36 (q, Me);
N-(7-Cyano-2,3-dioxo-1,2,3,4-tetrahydrochinoxalin-5-ylmethyl)-α-ethylamino-ethylphosphonsäure-hydrochlorid, Smp. > 270°C, MS(ES+): 353 (M+1).

Das Ausgangsmaterial kann beispielsweise folgendermassen erhalten werden:

### a) 7-Cyano-5-methyl-2,3-dimethoxy-chinoxalin

Eine Lösung von 7 g (24.72 mMol) 7-Brom-5-methyl-2,3-dimethoxy-chinoxalin (Beispiel 1 d), 1.74 g Zinkcyanid (14.83 mMol) und 1.1 g (0.9 mMol) Tetrakis(triphenylphosphine)-palladium(0) werden in 100 ml DMF gelöst, entgasst und unter Stickstofatmosphere gebracht. Anschliessend wird das Gemisch 16 Stunden auf 80°C erhitzt. Nach Abkühlen wird das Reaktionsgemisch versetzt mit 2N Salzsäure und mit Essigsäureethylester extrahiert. Die kombinierte organisch Phasen wurden mit Sole gewasschen, getrocknet über Natriumsulfat und eingedampft.. Der Rückstand wird an Kieselgel mit
Essigsäureethylester/Hexan (9:1- 1:1) als Laufmittel; Smp: 179-180°C (Essigester/ Hexan);
1 H-NMR (250 MHz, CDCl3): δ = 7.94 (d, J=3, 1 H), 7.51 (d, J=3, 1 H), 4.18 (s, 3H), 4.16(s, 3H), 2.64 (s, 3H); MS(ES+): 230

### Beispiel 26: In analoger Weise wie in den Beispielen 1 bis 23 beschrieben können ausgehend von 7-Trifluoromethyl-5-methyl-1,4-dihydro-chinoxalin-2,3-dion auch die folgenden Verbindungen hergestellt werden:

(R)-N-(7-Trifluormethyl-2,3-dioxo-1,2,3,4-tetrahydrochinoxalin-5-ylmethyl)-α-amino-ethylphosphonsäure, [α]D = -19.6° (c = 1, MeOH);
(S)-N-(7-Trifluormethyl-2,3-dioxo-1,2,3,4-tetrahydrochinoxalin-5-ylmethyl)-α-amino-ethylphosphonsäure, [α]D = +17.7° (c = 1, MeOH);
(R)-N-(7-Trifluormethyl-2,3-dioxo-1,2,3,4-tetrahydrochinoxalin-5-ylmethyl)-α-(ethylamino)-ethylphosphonsäure, [α]_{D} = +74° (c = 0.1, H₂0), Smp. > 270°C;N-(7-Trifluormethyl-2,3-dioxo-1,2,3,4-tetrahydrochinoxalin-5-ylmethyl)-β-(ethylamino)-ethylphosphonsäure, Smp. = 230°C (Zers.);
(S)-N-(7-Trifluormethyl-2,3-dioxo-1,2,3,4-tetrahydrochinoxalin-5-ylmethyl)-a-(ethylamino)-ethylphosphonsäure, [α]_{D} = -78° (c = 0.1, H₂0), Smp. > 270°C, MS(ES-): 394 (M-1);
(R)-N-(7-Trifluormethyl-2,3-dioxo-1,2,3,4-tetrahydrochinoxalin-5-ylmethyl)-β-amino-propylphosphonsäure-hydrochlorid, Smp. = 282°C (Zers);
(S)-N-(7-Triftuormethyl-2,3-dioxo-1,2,3,4-tetrahydrochinoxalin-5-ylmethyl)-β-amino-propylphosphonsäure-hydrochlorid, Smp. = 281°C (Zers);
N-(7-Trifluormethyl-2,3-dioxo-l ,2,3,4-tetrahydrochinoxalin-5-ylmethyl)-aminomethylphosphonsäure, Smp. = 321-323°C (Zers.)

### Das Ausgangsmaterial kan beispielsweise folgendermassen hergestellt werden:

### a) N-(2-Methyl-4-trifluoromethyl-phenyl)-oxalamsäureethylester

Eine Lösung von 37.5 g (214 mMol) 2-Methyl-4-trifluormethyl-anilin (DE 2750170 A1) und 44.7 ml (321 mMol) Triethylamin in 750 ml Essigsäureethylester wird mittels ein Eisbad auf +3° C gekühlt. Ethyloxalylchlorid (26.2 ml, 235.5 mMol) wird langzam zugetropft, so dass die Temperatur nicht über +10°C hinausstieg. Anschliessend wird noch 2 Stunden ausgerührt. Das Gemisch wird mit Wasser versetzt, mit eine 10 prozentige wässrige Natriumhydrogencarbonatlösung und mit Sole gewasschen, über Natriumsulfat getrocknet und eingeengt.
Kristallisation des rohproduktes ergibt 52.75 g der Titelverbindung als weisse Kristalle. Smpt. 120-121 °C (Essigester/Hexan)
1H-NMR (300 MHz, CDCl3): δ = 8.98 (br s, 1 H), 8.29 (d, J=8, 1 H), 7.53 (d, J=8, 1 H), 7.49 (s, 1 H), 4.45 (q, J=7, 2H), 2.40 (s, 3H), 1.64 (t, J=7, 3H).

### b) N-(2-Methyl-6-nitro-4-trifluoromethyl-phenyl)-oxalamsäureethylester

Zur einer eisgekühltem Lösung von 32.6 g (118.6 mMol) N-(2-Methyl-4-trifluoromethylphenyl)-oxalamsäureethylester in konzentriertem Schwefelsäure werden 14.4 g (142.3 mMol) Kaliumnitrat in kleinen Portionen gegeben. Nach 1.5 stundigen rühren bei 0°C wird das Gemisch vorsichtig auf 900 g Eis gegossen. Die weisse Suspension wird mit Essigsäureethylester extrahiert. Die organische Phase wird mit Sole gewasschen, über Natriumsulfat getrocknet und unter verminderten Druck einrotiert. Kristallisation aus Essigester/Hexan ergibt 35.5 g der Titelverbindung als weisse Kristalle.
Smpt.: 118-120°C.
1 H-NMR (200 MHz, CDCl3): δ = 9.94 (br s, NH), 8.17 (s, 1 H), 7.81 (s, 1H), 4.45 (q, J=7, 2H), 2.41 (s, 3H), 1.43 (t, J=7, 3H).

### c) 7-Trifluoromethyl-5-methyl-1,4-dihydro-chinoxalin-2,3-dion

Unter einer Stickstoffatmosphäre bei 0°C werden 355 ml einer 15-prozentiger Titantrichloridlösung in wässriger salzsäure gelöst in 850 ml Wasser und 850 ml Aceton. Einer Lösung von 35.5 g (110.8 mMol) N-(2-Methyl-6-nitro-4-trifluoromethyl-phenyl)-oxalamsäureethylester in 1.7 L Aceton wird langsam dazugetropft. Die entsandene violette Lösung wird 16 Stunden beo 0°C gerührt. Anschliessend wird so viel 15-prozentiger Titantrichloridiösung in wässriger salzsäure zugegeben, bis gemäss 1 H-NMR Analyse kein Edukt mehr nachweisbar war. Das Reaktionsgemisch wird abgenutscht, das Filtrat eingeengt und die ausgefallene Feststoff wieder abgenutscht. Das Rohprodukt wird gewasschen mit verdünte Salzsäure und Wasser. Der Titelverbindung wird als weisser Feststoff erhalten.
MS(ES⁺): 245 (M+H)⁺
1 H-NMR (200 MHz, DMSO-d6): δ = 12.1 (s, NH), 11.5 (s, NH), 7.30 (s, 1 H), 7.28 (s, 1 H), 2.40 (s, 3H).

### Beispiel 27: Tabletten, enthaltend je 50 mg N-(2,3-Dioxo-7-nitro-1,2,3,4-tetrahydrochinoxalin-5-ylmethyl)-α-amino-(isopropyl)phosphonsäure oder ein Salz, z.B. das Hydrobromid, davon können wie folgt hergestellt werden:

**Zusammensetzung (10000 Tabletten)**

| | |
|---|---|
| Wirkstoff | 500.0 g |
| Laktose | 500.0 g |
| Kartoffelstärke | 352.0 g |
| Gelatine | 8.0 g |
| Talk | 60.0 g |
| Magnesiumstearat | 10.0 g |
| Siliciumdioxid (hochdispers) | 20.0 g |
| Ethanol | q.s. |

Der Wirkstoff wird mit der Laktose und 292 g Kartoffelstärke vermischt, die Mischung mit einer ethanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 28: Eine sterilfiltrierte wässrige Gelatine-Lösung mit 20 % Cyclodextrinen als Lösungsvermittler, enthaltend je 3 mg N-(2,3-Dioxo-7-nitro-1,2,3,4-tetrahydrochinoxalin-5-ylmethyl)-α-amino-(isopropyl)phosphonsäure oder ein Salz, z.B. das Hydrobromid, davon als Wirkstoff wird unter Erwärmen mit einer sterilen Gelatinelösung, welche als Konservierungsmittel Phenol enthält, unter aseptischen Bedingungen so vermischt, dass 1.0 ml Lösung die folgende Zusammensetzung hat:

| | |
|---|---|
| Wirkstoff | 3 mg |
| Gelatine | 150.0 mg |
| Phenol | 4.7 mg |
| dest. Wasser mit 20 % Cyclodextrinen als Lösungsvermittler | 1.0 ml |

Beispiel 29: Zur Herstellung einer sterilen Trockensubstanz zur Injektion, enthaltend je 5 mg N-(2,3-Dioxo-7-nitro-1,2,3,4-tetrahydrochinoxalin-5-ylmethyl)-α-amino-(isopropyl)-phosphonsäure oder ein Salz, z.B. das Hydrobromid, davon löst man 5 mg einer der in den vorausgehenden Beispielen genannten Verbindungen der Formel I als Wirkstoff in 1 ml einer wässrigen Lösung mit 20 mg Mannit und 20 % Cyclodextrinen als Lösungsvermittler. Die Lösung wird sterilfiltriert und unter aseptischen Bedingungen in eine 2 ml-Ampulle gefüllt, tiefgekühlt und lyophilisiert. Vor dem Gebrauch wird das Lyophilisat in 1 ml destilliertem Wasser oder 1 ml physiologischer Kochsalzlösung gelöst. Die Lösung wird intramuskulär oder intravenös angewendet. Diese Formulierung kann auch in Doppelkammerspritzampullen abgefüllt werden.

Beispiel 30: Für die Herstellung von 10 000 Lacktabletten, enthaltend je 100 mg N-(2,3-Dioxo-7-nitro-1,2,3,4-tetrahydrochinoxalin-5-ylmethyl)-α-amino-(isopropyl)phosphonsäure oder ein Salz, z.B. das Hydrobromid, davon, können wie folgt hergestellt werden:

| | |
|---|---|
| Wirkstoff | 1000 g |
| Maisstärke | 680 g |
| Kolloidale Kieselsäure | 200 g |
| Magnesiumstearat | 20 g |
| Stearinsäure | 50 g |
| Natriumcarboxymethylstärke | 250 g |
| Wasser | q. s. |

Ein Gemisch von einer der in den vorausgehenden Beispielen genannten Verbindungen der Formel 1 als Wirkstoff, 50 g Maisstärke und der kolloidalen Kieselsäure wird mit Stärkekleister aus 250 g Maisstärke und 2.2 kg entmineralisiertem Wasser zu einer feuchten Masse verarbeitet. Diese wird durch ein Sieb von 3 mm Maschenweite getrieben und bei 45° während 30 Minuten im Wirbelschichttrockner getrocknet. Das getrocknete Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

Beispiel 31: In analoger Weise wie in den Beispielen 27 bis 30 beschrieben kann man ferner pharmazeutische Präparate enthaltend eine andere Verbindung gemäss einem der Beispielen 1 bis 26 herstellen.

## Patentansprüche

1. Eine Verbindung ausgewählt aus
N-Acetyl-N-(7-brom-2,3-dioxo-1,2,3,4-tetrahydrochinoxalin-5-ylmethyl)-aminomethylphosphonsäure;
N-Acetyl-N-(7-chlor-2,3-dioxo-1,2,3,4-tetrahydrochinoxalin-5-ylmethyl)-aminomethylphosphonsäure;
N-Acetyl-N-(7-fluor-2,3-dioxo-1,2,3,4-tetrahydrochinoxalin-5-ylmethyl)-aminomethylphosphonsäure;
N-(2,3-Dioxo-7-nitro-1,2,3,4-tetrahydrochinoxalin-5-ylmethyl)-α-(ethylamino)-ethylphosphonsäure;
N-(7-Chlor-2,3-dioxo-1,2,3,4-tetrahydrochinoxalin-5-ylmethyl)-α-(methylamino)-ethylphosphonsäure;
N-(7-Fluor-2,3-dioxo-1,2,3,4-tetrahydrochinoxalin-5-ylmethyl)-α-(ethylamino)-ethylphosphonsäure;
N-(2,3-Dioxo-7-nitro-1,2,3,4-tetrahydrochinoxalin-5-ylmethyl)-α-(methylamino)-ethylphosphonsäure;
N-(2,3-Dioxo-7-nitro-1,2,3,4-t etrahydrochinoxalin-5-ylmethyl)-β-amino-propylphosphonsäure;
N-(2,3-Dioxo-7-nitro-1,2,3,4-tetrahydrochinoxalin-5-ylmethyl)-2-amino-phenylphosphonsäure;
N-(7-Brom-2,3-dioxo-1,2,3,4-tetrahydrochinoxalin-5-ylmethyl)-α-aminobenzyl-phosphonsäure;
N-(7-Bromo-2,3-dioxo-1,2,3,4-tetrahydrochinoxalin-5-ylmethyl)-α-amino-3-methylbutyl-phosphonsäure;N-(7-Bromo-2,3-dioxo-1,2,3,4-tetrahydrochinoxalin-5-ylmethyl)-α-amino-β-benzyloxyethylphosphonsäure;
N-(7-Bromo-2,3-dioxo-1,2,3,4-tetrahydrochinoxalin-5-ylmethyl)-α-amino-propylphosphonsäure;
N-(2,3-Dioxo-7-nitro-1,2,3,4-tetrahydrochinoxalin-5-ylmethyl)-4-amino-benzyl-phosphonsäurediethylesterN-(2,3-Dioxo-7-nitro-1,2,3,4-tetrahydrochinoxalin-5-ylmethyl)-α-amino-(3-hydroxybenzyl)-phosphonsäure;
N-(2,3-Dioxo-7-nitro-1,2,3,4-tetrahydrochinoxalin-5-ylmethyl)-α-amino-(isopropyl)-phosphonsäure;
N-(2,3-Dioxo-7-nitro-1,2,3,4-tetrahydrochinoxalin-5-ytmethyl)-4-amino-benzyl-phosphonsäure;trans-2-[N-(2,3-Dioxo-7-nitro-1,2,3,4-tetrahydrochinoxalin-5-ylmethyl)-amino]-cyclopropylphosphonsäure;
N-(2,3-Dioxo-7-nitro-1,2,3,4-tetrahydrochinoxalin-5-ylmethyl)α-amino-(3,4,5-trimethoxybenzyl)-phosphonsäure;
N-(2,3-Dioxo-7-nitro-1,2,3,4-tetrahydrochinoxalin-5-ylmethyl)-α-amino-cyclohexylmethyl-phosphonsäure:
N-(2,3-Dioxo-7-nitro-1,2,3,4-tetrahydrochinoxalin-5-ylmethyl)-α-amino-*n*-butyl-phosphonsäure;
N-(2,3-Dioxo-7-nitro-1,2,3,4-tetrahydrochinoxalin-5-ylmethyl)-α-amino-3-methylbutyl-phosphonsäure;
N-(2,3-Dioxo-7-nitro-1,2,3,4-tetrahydrochinoxalin-5-ylmethyl)-α-amino-benzyl-phosphonsäure;
N-(2,3-Dioxo-7-nitro-1,2,3,4-tetrahydrochinoxalin-5-ylmethyl)-α-amino-(3-thienyl)-methylphosphonsäure;
N-(2,3-Dioxo-7-nitro-1,2,3,4-tetrahydrochinoxalin-5-ylmethyl)-α-amino-(4-methoxycarbonylbenzyl)-phosphonsäure;
N-(2,3-Dioxo-7-nitro-1,2,3,4-tetrahydrochinoxalin-5-ylmethyl)-α-amino-(3-nitrobenzyl)-phosphonsäure;
-(2,3-Dioxo-7-nitro-1,2,3,4-tetrahydrochinoxalin-5-ylmethyl)-α-amino-(2-hydroxy-3-methoxybenzyl)-phosphonsäure;
N-(7-Chlor-2,3-dioxo-1,2,3,4-tetrahydrochinoxalin-5-ylmethyl)-2-aminophenylphosphonsäure;
N-(7-Nitro-2,3-dioxo-1,2,3,4-tetrahydrochinoxalin-5-ylmethyl)-2-aminophenylphosphonsäure;
N-(7-Chlor-2,3-dioxo-1,2,3,4-tetrahydrochinoxalin-5-ylmethyl)-α-amino-ethylphosphonsäure ;
N-(7-Fluor-2,3-dioxo-1,2,3,4-tetrahydrochinoxalin-5-ylmethyl)-α-amino-ethylphosphonsäure;
P-Benzyl N-(2,3-dioxo-7-nitro-1,2,3,4-tetrahydrochinoxalin-5-ylmethyl)-aminomethylphosphinsäure;
P-Methyl-N-(2,3-dioxo-7-nitro-1,2,3,4-tetrahydrochinoxalin-5-ylmethyl)-α-aminoethylphosphinsäure;
P-Benzyl-(7-nitro-2,3-dioxo-1,2,3,4-tetrahydrochinoxalin-5-yl)-aminomethanphosphinsäure;
P-Methyl-(7-nitro-2,3-dioxo-1,2,3,4-tetrahydrochinoxalin-5-yl)-aminomethanphosphinsäure;
N-(7-Nitro-2,3-dioxo-1,2,3,4-tetrahydrochinoxalin-5-ylmethyl)-4-phosphono-buttersäureamid;
N-(7-Nitro-2,3-dioxo-1,2,3,4-tetrahydrochinoxalin-5-ylmethyl)-2-phosphono-essigsäureamid;
N-(7-Nitro-2,3-dioxo-1,2,3,4-tetrahydrochinoxalin-5-ylmethyl)-3-phosphonopropensäureamid;
N-(7-Nitro-2,3-dioxo-1,2,3,4-tetrahydrochinoxalin-5-ylmethyl)-2-phosphono-indan-2-carbonsäureamid;
N-Benzyl-N-(2,3-dioxo-7-nitro-1,2,3,4-tetrahydrochinoxalin-5-ylmethyl)-amino-methylphosphonsäure;
N-Benzyl-N-(7-brom-2,3-dioxo-1,2,3,4-tetrahydrochinoxalin-5-ylmethyl)-amino-methylphosphonsäure;
N-Benzyl-N-(7-chlor-2,3-dioxo-1,2,3,4-tetrahydrochinoxalin-5-ylmethyl)-amino-methylphosphonsäure;
N-Benzyl-N-(2,3-dioxo-7-fluor-1,2,3,4-tetrahydrochinoxalin-5-ylmethyl)-amino-methylphosphonsäure;
N-Benzyl-N-(2,3-dioxo-7-nitro-1,2,3,4-tetrahydrochinoxalin-5-ylmethyl)-α-aminoethanphosphonsäure;
N-(2,3-Dioxo-7-fluor-1,2,3,4-tetrahydrochinoxalin-5-ylmethyl)-α-aminoethylphosphonsäure;
N-(2,3-Dioxo-7-nitro-1,2,3,4-tetrahydro-chinoxalin-5-ylmethyl)-α-amino-propylphosphonsäure;
N-(2,3-Dioxo-7-nitro-1,2,3,4-tetrahydro-chinoxalin-5-ylmethyl)-α-amino-(tetrahydropyran-4-yl)-phosphonsäure;
N-(2,3-Dioxo-7-nitro-1,2,3,4-tetrahydro-chinoxalin-5-ylmethyl)-α-amino-(piperidin-4-yl)-phosphonsäure;
N-(2,3-Dioxo-7-nitro-1,2,3,4-tetrahydro-chinoxalin-5-ylmethyl)-α-amino-(furan-2-ylmethyl)-phosphonsäure;
N-(2,3-Dioxo-7-nitro-1,2,3,4-tetrahydro-chinoxalin-5-ylmethyl)-α-amino-(2-methoxy)-ethylphosphonsäure;
N-(2,3-Dioxo-7-nitro-1,2,3,4-tetrahydro-chinoxalin-5-ylmethyl)-2-amino-2-cyclohexyl-ethylphosphonsäure;
N-(2,3-Dioxo-7-nitro-1,2,3,4-tetrahydro-chinoxalin-5-ylmethyl)-β-amino-isopropyl-phosphonsäure;
N-(2,3-Dioxo-7-nitro-1,2,3,4-tetrahydro-chinoxalin-5-ylmethyl)-2-amino-cyclohexyl-phosphonsäure;
N-(2,3-Dioxo-7-nitro-1,2,3,4-tetrahydro-chinoxalin-5-ylmethyl)-2-amino-2-methyl-propylphosphonsäure;
N-(2,3-Dioxo-7-nitro-1,2,3,4-fetrahydro-chinoxalin-5-ylmethyl)-2-amino-butyl-phosphonsäure;
N-(2,3-Dioxo-7-nitro-1,2,3,4-tetrahydro-chinoxalin-5-ylmethyl)-2-amino-heptyl-phosphonsäure;
N-(2,3-Dioxo-7-nitro-1,2,3,4-tetrahydro-chinoxalin-5-ylmethyl)-2-amino-3-phenyloxy-propylphosphonsäure;
N-(2,3-Dioxo-7-nitro-1,2,3,4-tetrahydro-chinoxalin-5-ylmethyl)-2-amino-2-p-tolyl-ethylphosphonsäure;
N-(2,3-Dioxo-7-nitro-l ,2.3,4-tetrahydro-chinoxalin-5-ylmethyl)-2-amino-2-(2-methoxy-phenyl)-ethylphosphonsäure;
N-(2,3-Dioxo-7-nitro-1,2,3,4-tetrahydro-chinoxalin-5-ylmethyl)-2-amino-2-(4-fluor-phenyl)-ethylphosphonsäure;
N-(2,3-Dioxo-7-nitro-1,2,3,4-tetrahydro-chinoxalin-5-ylmethyl)-2-amino-2-phenyl-ethylphosphonsäure;
N-(2,3-Dioxo-7-nitro-1,2,3,4-tetrahydro-chinoxalin-5-ylmethyl)-2-amino-1-(furan-2-yl)-ethylphosphonsäure;
N-(2,3-Dioxo-7-nitro-1,2,3,4-tetrahydro-chinoxalin-5-ylmethyl)-2-amino-1-(4-fluor-phenyl)-ethylphosphonsäure;
N-(2,3-Dioxo-7-nitro-1,2,3,4-tetrahydro-chinoxalin-5-ylmethyl)-2-amino-1-(4-mothoxy-phenyl)-ethylphosphonsäure;
N-(2,3-Dioxo-7-nitro-1,2,3,4-tetrahydro-chinoxalin-5-ylmethyl)-2-amino-1-(3-methoxy-phenyl)-ethylphosphonsäure;
N-(2,3-Dioxo-7-nitro-1,2,3,4-tetrahydro-chinoxalin-5-ylmethyl)-2-amino-1-(2-chlor-phenyl)-ethylphosphonsäure;
N-(2,3-Dioxo-7-nitro-1,2,3,4-tetrahydro-chinoxalin-5-ylmethyl)-2-amino-1-(4-tolyl)-ethylphosphonsäure:
N-Benzyl-N-(7-brom-2,3-dioxo-1,2,3,4-tetrahydrochinoxalin-5-ylmethyl)-α-amino-ethylphosphonsäure;
N-Benzyl-N-(7-chlor-2,3-dioxo-1,2,3,4-tetrahydrochinoxalin-5-ylmethyl)-α-amino-ethylphosphonsäure und
N-Benzyl-N-(2,3-dioxo-7-fluor-1,2,3,4-tetrahydrochinoxalin-5-ylmethyl)-α-amino-ethylphosphonsäure;
und Salzen davon.

2. Eine Verbindung gemäss Anspruch 1 in freier Form oder in einer pharmazeutisch verwendbaren Salzform zur Anwendung inenem Verfahren zur therapeutischen Behandlung des menschlichen oder tierischen Körpers.

3. Pharmazeutische Präparate, enthaltend eine Verbindung gemäß Anspruch 1 oder ein pharmazeutisch verwendbares Salz davon neben üblichen pharmazeutischen Hilfs- und Trägerstoffen.

4. Verwendung einer Verbindung gemäss Anspruch 1 Herstellung eines Arzneimittels zur Behandlung von pathologischen Zuständen, die auf Blockierung von excitatorischen Aminosäurerezeptoren ansprechen.

5. Verwendung einer Verbindung gemäss Anspruch 1 zur Herstellung eines Arzneimittels zur Behandlung von neurodegenerativen Erkrankungen, ischämischen Hirnerkrankungen, Gefäss-und Muskelspasmen, Konvulsionen und Angst-und Schmerzzustanden.

6. Verwendung gemäβ Anspruch 5 zur Behandlung von Epilepsie.

## Claims

1. A compound selected from
N-acetyl-N-(7-bromo-2,3-dioxo-1,2,3,4-tetrahydroquinoxalin-5-ylmethyl)-aminomethylphosphonic acid;
N-acetyl-N-(7-chloro-2,3-dioxo-1,2,3,4-tetrahydroquinoxalin-5-ylmethyl)-aminomethylphosphonic acid;
N-acetyl-N-(7-fluoro-2, 3-dioxo-1, 2, 3,4-tetrahydroquinoxalin-5-ylmethyl)-aminomethylphosphonic acid;
N-(2,3-dioxo-7-nitro-1,2,3,4-tetrahydroquinoxalin-5-ylmethyl)-α-(ethylamino)-ethylphosphonic acid;
N-(7-chloro-2,3-dioxo-1,2,3,4-tetrahydroquinoxalin-5-ylmethyl)-α-(methylamino)-ethylphosphonic acid;
N-(7-fluoro-2,3-dioxo-1,2,3,4-tetrahydroquinoxalin-5-ylmethyl)-a-(ethylamino)-ethylphosphonic acid;
N-(2,3-dioxo-7-nitro-1,2,3,4-tetrahydroquinoxalin-5-ylmethyl)-α-(methylamino)-ethylphosphonic acid;
N-(2,3-dioxo-7-nitro-1,2,3,4-tetrahydroquinoxalin-5-ylmethyl)-13-amino-propyl-phosphonic acid;
N-(2,3-dioxo-7-nitro-1,2,3,4-tetrahydroquinoxalin-5-ylmethyl)-2-amino-phenyl-phosphonic acid;
N-(7-bromo-2,3-dioxo-1,2,3,4-tetrahydroquinoxalin-5-ylmethyl)-α-aminobenzyl-phosphonic acid;
N-(7-bromo-2,3-dioxo-1,2,3,4-tetrahydroquinoxalin-5-ylmethyl)-α-amino-3-methylbutyl-phosphonic acid;
N-(7-bromo-2,3-dioxo-1,2,3,4-tetrahydroquinoxalin-5-ylmethyl)-α-amino-β-benzyloxyethylphosphonic acid;
N-(7-bromo-2,3-dioxo-1,2,3,4-tetrahydroquinoxalin-5-ylmethyl)-α-amino-propyl-phosphonic acid;
N-(2,3-dioxo-7-nitro-1,2,3,4-tetrahydroquinoxalin-5-ylmethyl)-4-amino-benzyl-phosphonic acid diethylester;
N-(2,3-dioxo-7-nitro-1,2,3,4-tetrahydroquinoxalin-5-ylmethyl)-α-amino-(3-hydroxybenzyl)-phosphonic acid;
N-(2,3-dioxo-7-nitro-1,2,3,4-tetrahydroquinoxalin-5-ylmethyl)-α-amino-(isopropyl)-phosphonic acid;
N-(2,3-dioxo-7-nitro-1,2,3,4-tetrahydroquinoxalin-5-ylmethyl)-4-amino-benzyl-phosphonic acid;
*trans*-2-[N-(2,3-dioxo-7-nitro-1,2,3,4-tetrahydroquinoxalin-5-ylmethyl)-amino]-cyclopropyl-phosphonic acid;
N-(2,3-dioxo-7-nitro-1,2,3,4-tetrahydroquinoxalin-5-ylmethyl)-α-amino-(3,4,5-trimethoxybenzyl)-phosphonic acid;
N-(2,3-dioxo-7-nitro-1,2,3,4-tetrahydroquinoxalin-5-ylmethyl)-α-amino-cyclohexylmethyl-phosphonic acid;
N-(2,3-dioxo-7-nitro-1,2,3,4-tetrahydroquinoxalin-5-ylmethyl)-α-amino-*n*-butyl-phosphonic acid;
N-(2,3-dioxo-7-nitro-1,2,3,4-tetrahydroquinoxalin-5-ylmethyl)-α-amino-3-methylbutyl-phosphonic acid;
N-(2,3-dioxo-7-nitro-1,2,3,4-tetrahydroquinoxalin-5-ylmethyl)-α-amino-benzyl-phosphonic acid;
N-(2,3-dioxo-7-nitro-1,2,3,4-tetrahydroquinoxalin-5-ylmethyl)-α-amino-(3-thienyl)-methylphosphonic acid;
N-(2,3-dioxo-7-nitro-1,2,3,4-tetrahydroquinoxalin-5-ylmethyl)-α-amino-(4-methoxycarbonylbenzyl)-phosphonic acid;
N-(2,3-dioxo-7-nitro-1,2,3,4-tetrahydroquinoxalin-5-ylmethyl)-α-amino-(3-nitrobenzyl)-phosphonic acid;
-(2,3-dioxo-7-nitro-1,2,3,4-tetrahydroquinoxalin-5-ylmethyl-α-amino-(2-hydroxy-3-methoxybenzyl)-phosphonic acid;
N-(7-chloro-2,3-dioxo-1,2,3,4-tetrahydroquinoxalin-5-ylmethyl)-2-aminophenyl-phosphonic acid;
N-(7-nitro-2,3-dioxo-1,2,3,4-tetrahydroquinoxalin-5-ylmethyl)-2-aminophenyl-phosphonic acid;
N-(7-chloro-2,3-dioxo-1,2,3,4-tetrahydroquinoxalin-5-ylmethyl)-α-amino-ethyl-phosphonic acid;
N-(7-fluoro-2,3-dioxo-1,2,3,4-tetrahydroquinoxalin-5-ylmethyl)-α-amino-ethyl-phosphonic acid;
P-benzyl-N-(2,3-dioxo-7-nitro-1,2,3,4-tetrahydroquinoxalin-5-ylmethyl)-aminomethyl-phosphinic acid;
P-methyl-N-(2,3-dioxo-7-nitro-1,2,3,4-tetrahydroquinoxalin-5-ylmethyl)-α-aminoethyl-phosphinic acid;
P-benzyl-(7-nitro-2,3-dioxo-1,2,3,4-tetrahydroquinoxalin-5-yl)-aminomethane-phosphinic acid;
P-methyl-(7-nitro-2,3-dioxo-1,2,3,4-tetrahydroquinoxalin-5-yl)-aminomethane-phosphinic acid;
N-(7-nitro-2,3-dioxo-1,2,3,4-tetrahydroquinoxalin-5-ylmethyl)-4-phosphono-butyric acid amide;
N-(7-nitro-2,3-dioxo-1,2,3,4-tetrahydroquinoxalin-5-ylmethyl)-2-phosphono-acetic acid amide;
N-(7-nitro-2.3-dioxo-1,2,3,4-tetrahydroquinoxalin-5-ylmethyl)-3-phosphono-propenoic acid amide;
N-(7-nitro-2,3-dioxo-1,2,3,4-tetrahydroquinoxalin-5-ylmethyl)-2-phosphono-indane-2-carboxylic acid amide;
N-benzyl-N-(2,3-dioxo-7-nitro-1,2,3,4-tetrahydroquinoxalin-5-ylmethyl)-amino-methyl-phosphonic acid;
N-benzyl-N-(7-bromo-2,3-dioxo-1,2,3,4-tetrahydroquinoxalin-5-ylmethyl)-amino-methyl-phosphonic acid;
N-benzyl-N-(7-chloro-2,3-dioxo-1,2,3,4-tetrahydroquinoxalin-5-ylmethyl)-amino-methyl-phosphonic acid;
N-benzyl-N-(2,3-dioxo-7-fluoro-1,2,3,4-tetrahydroquinoxalin-5-ylmethyl)-amino-methyl-phosphonic acid;
N-benzyl-N-(2,3-dioxo-7-nitro-1,2,3,4-tetrahydroquinoxalin-5-ylmethyl)-α-amino-ethane-phosphonic acid;
N-(2,3-dioxo-7-fluoro-1,2,3,4-tetrahydroquinoxalin-5-ylmethyl)-α-amino-ethyl-phosphonic acid;
N-(2,3-dioxo-7-nitro-1,2,3,4-tetrahydroquinoxalin-5-ylmethyl)-α-amino-propyl-phosphonic acid;
N-(2,3-dioxo-7-nitro-1,2,3,4-tetrahydro-quinoxalin-5-ylmethyl)-α-amino-(tetrahydropyran-4-yl)-phosphonic acid;
N-(2,3-dioxo-7-nitro-1,2,3,4-tetrahydro-quinoxalin-5-ylmethyl)-α-amino-(piperidin-4-yl)-phosphonic acid;
N-(2,3-dioxo-7-nitro-1,2,3,4-tetrahydro-quinoxalin-5-ylmethyl)-α-amino-(furan-2-ylmethyl)-phosphonic acid;
N-(2,3-dioxo-7-nitro-1,2,3,4-tetrahydro-quinoxalin-5-ylmethyl)-α-amino-(2-methoxy)-ethylphosphonic acid;
N-(2,3-dioxo-7-nitro-1,2,3,4-tetrahydro-quinoxalin-5-ylmethyl)-2-amino-2-cyclohexyl-ethylphosphonic acid;
N-(2,3-dioxo-7-nitro-1,2,3,4-tetrahydroquinoxalin-5-ylmethyl)-β-amino-isopropyl-phosphonic acid;
N-(2,3-dioxo-7-nitro-1,2,3,4-tetrahydro-quinoxalin-5-ylmethyl)-2-amino-cyclohexyl-phosphonic acid;
N-(2,3-dioxo-7-nitro-1,2,3,4-tetrahydro-quinoxalin-5-ylmethyl)-2-amino-2-methyl-propyl-phosphonic acid;
N-(2,3-dioxo-7-nitro-1,2,3,4-tetrahydro-quinoxalin-5-ylmethyl)-2-amino-butyl-phosphonic acid;
N-(2,3-dioxo-7-nitro-1,2,3,4-tetrahydro-quinoxalin-5-ylmethyl)-2-amino-heptyl-phosphonic acid;
N-(2,3-dioxo-7-nitro-1,2,3,4-tetrahydro-quinoxalin-5-ylmethyl)-2-amino-3-phenyloxy-propyl-phosphonic acid;
N-(2,3-dioxo-7-nitro-1,2,3,4-tetrahydro-quinoxalin-5-ylmethyl)-2-amino-2-p-tolyl-ethylphosphonic acid;
N-(2,3-dioxo-7-nitro-1,2,3,4-tetrahydro-quinoxalin-5-ylmethyl)-2-amino-2-(2-methoxyphenyl)-ethyl-phosphonic acid;
N-(2,3-dioxo-7-nitro-1,2,3,4-tetrahydro-quinoxatin-5-ylmethyl)-2-amino-2-(4-fluorophenyl)-ethyl-phosphonic acid;
N-(2,3-dioxo-7-nitro-1,2,3,4-tetrahydro-quinoxalin-5-ylmethyl)-2-amino-2-phenyl-ethylphosphonic acid;
N-(2,3-dioxo-7-nitro-1,2,3,4-tetrahydro-quinoxalin-5-ylmethyl)-2-amino-1-(furan-2-yl)-ethylphosphonic acid;
N-(2,3-dioxo-7-nitro-1,2,3,4-tetrahydro-quinoxalin-5-ylmethyl)-2-amino-1-(4-fluorophenyl)-ethyl-phosphonic acid;
N-(2,3-dioxo-7-nitro-1,2,3,4-tetrahydro-quinoxalin-5-ytmethyl)-2-amino-1-(4-methoxy-phenyl)-ethyl-phosphonic acid;
N-(2,3-dioxo-7-nitro-1,2,3,4-tetrahydro-quinoxalin-5-ylmethyl)-2-amino-1-(3-methoxy-phenyl)-ethyl-phosphonic acid;
N-(2,3-dioxo-7-nitro-1,2,3,4-tetrahydro-quinoxalin-5-ylmethyl)-2-amino-1-(2-chloro-phenyl)-ethyl-phosphonic acid;
N-(2,3-dioxo-7-nitro-1,2,3,4-tetrahydro-quinoxalin-5-ylmethyl)-2-amino-1-(4-tolyl)-ethylphosphonic acid;
N-benzyl-N-(7-bromo-2,3-dioxo-1,2,3,4-tetrahydroquinoxalin-5-ylmethyl)-α-amino-ethylphosphonic acid;
N-benzyl-N-(7-chloro-2,3-dioxo-1,2,3,4-tetrahydroquinoxalin-5-ylmethyl)-α-amino-ethylphosphonic acid and
N-benzyl-N-(2,3-dioxo-7-fluoro-1,2,3,4-tetrahydroquinoxalin-5-ylmethyl)-α-amino-ethylphosphonic acid;
and salts thereof.

2. A compound according to claim 1 in free form or in a pharmaceutically acceptable salt form, for use in a process for the therapeutic treatment of humans or animals.

3. Pharmaceutical preparations, containing a compound according to claim 1 or a pharmaceutically acceptable salt thereof, together with conventional pharmaceutical excipients and carriers.

4. Use of a compound according to claim 1, in the production of a medicament for the treatment of pathological conditions which respond to the blocking of excitatory amino acid receptors.

5. Use of a compound according to claim 1, in the production of a medicament for the treatment of neurodegenerative diseases, ischaemic brain disorders, angiospasms and muscular spasms, convulsions, and anxiety and pain conditions.

6. Use according to claim 5 in the treatment of epilepsy.

## Revendications

1. Composé choisi parmi
l'acide N-acétyl-N-(7-bromo-2,3-dioxo-1,2,3,4-tétra-hydroquinoxaline-5-ylméthyl)-aminométhylphosphonique ;
l'acide N-acétyl-N-(7-chloro-2,3-dioxo-1,2,3,4-tétrahydroquinoxaline-5-ylméthyl)-aminométhyl-phosphonique ;
l'acide N-acétyl-N-(7-fluoro-2,3-dioxo-1,2,3,4-tétrahydroquinoxaline-5-ylméthyl)-aminométhyl-phosphonique ;
l'acide N-(2,3-dioxo-7-nitro-1,2,3,4-tétrahydro-quinoxaline-5-ylméthyl)-α (éthylamino)-éthyl-phosphonique ;
l'acide N-(7-chloro-2,3-dioxo-1,2,3,4-tétrahydro-quinoxaline-5-ylméthyl)-α-(méthylamino)-éthyl-phosphonique ;
l'acide N-(7-fluoro-2,3-dioxo-1,2,3,4-tétrahydro-quinoxaline-5-ylméthyl)-α-(éthylamino)-éthyl-phosphonique ;
l'acide N-(2,3-dioxo-7-nitro-1,2,3,4-tétrahydro-quinoxaline-5-ylméthyl)-α-(méthylamino)-éthyl-phosphonique ;
l'acide N-(2,3-dioxo-7-nitro-1,2,3,4-tétrahydro-quinoxaline-5-ylméthyl)-β-amino-propylphosphonique ;
l'acide N-(2,3-dioxo-7-nitro-1,2,3,4-tétrahydro-quinoxaline-5-ylméthyl)-2-amino-phénylphosphonique ;
l'acide N-(7-bromo-2,3-dioxo-1,2,3,4-tétrahydro-quinoxaline-5-ylinéthyl)-α-aminobenzylphosphonique ;
l'acide N-(7-bromo-2,3-dioxo-1,2,3,4-tétrahydro-quinoxaline-5-ylméthyl)-α-amino-3-méthylbutyl-phosphonique ; acide N-(7-bromo-2,3-dioxo-1,2,3,4-tétrahydro-quinoxaline-5-ylméthyl)-α-amino-β-benzyloxyéthylphosphonique ;
l'acide N-(7-bromo-2,3-dioxo-1,2,3,4-tétrahydro-quinoxaline-5-ylméthyl)-α-amino-propyl-phosphonique ;
l'acide N-(2,3-dioxo-7-nitro-1,2,3,4-tétrahydro-quinoxaline-5-ylméthyl)-4-amino-benzyl-phosphonique diéthylester ; acide N-(2,3-dioxo-7-nitro-1,2,3,4-tétrahydro-quinoxaline-5-ylméthyl)-α-amino-(3-hydroxybenzyl)-phosphonique ;
l'acide N-(2,3-dioxo-7-nitro-1,2,3,4-tétrahydro-quinoxaline-5-ylméthyl)-α-amino-(isopropyl)-phosphonique ;
l'acide N-(2,3-dioxo-7-nitro-1,2,3,4-tétrahydro-quinoxaline-5-ylméthyl)-4-amino-benzyl-phosphonique trans acide 2-[N-(2,3-dioxo-7-nitro-1,2,3,4-tétrahydro-quinoxaline-5-ylméthyl)-amino]-cyclopropylphosphonique ;
l'acide N-(2,3-dioxo-7-nitro-1,2,3,4-tétrahydro-quinoxaline-5-ylméthyl)-α-amino-(3,4,5-triméthoxy-benzyl)-phosphonique ;
l'acide N-(2,3-dioxo-7-nitro-1,2,3,4-tétrahydro-quinoxaline-5-ylméthyl)-α-amino-cyclohexylméthyl-phosphonique;
l'acide N-(2,3-dioxo-7-nitro-1,2,3,4-tétrahydro-quinoxaline-5-ylméthyl)-α-amino-n-butyl-phosphonique ;
l'acide N-(2,3-dioxo-7-nitro-1,2,3,4-tétrahydro-quinoxaline-5-ylméthyl)-α amino-3-méthylbutyl-phosphonique ;
l'acide N-(2,3-dioxo-7-nitro-1,2,3,4-tétrahydro-quinoxaline-5-ylméthyl)-α amino-benzyl-posphonique ;
l'acide N-(2,3-dioxo-7-nitro-1,2,3,4-tétrahydro-qumoxaline-5-ylméthyl)-α-amino-(3-thiényl)-méthyl-phosphonique ;
l'acide N-(2,3-dioxo-7-nitro-1,2,3,4-tétrahydro-quinoxaline-5-ylméthyl)-α-amino-(4-méthoxycarbonyl-benzyl)-phosphonique ;
l'acide N-(2,3-dioxo-7-nitro-1,2,3,4-tétrahydro-quinoxaline-5-ylméthyl)-α amino-(3-nitrobenzyl)-phosphonique ;
l'acide N-(2,3-dioxo-7-nitro-1,2,3,4-tétrahydro-quinoxaline-5-ylméthyl)-α-amino-(2-hydroxy-3-méthoxy-benzyl)-phosphonique ;
l'acide N-(7-chloro-2,3-dioxo-1,2,3,4-tétrahydro-quinoxaline-5-ylméthyl)-2-aminophényl-phosphonique ;
l'acide N-(7-nitro-2,3-dioxo-1,2,3,4-tétrahydro-quinoxaline-5-ylméthyl)-2-aminophényl-phosphonique ;
l'acide N-(7-chloro-2,3-dioxo-1,2,3,4-tétrahydro-quinoxaline-5-ylméthyl)-α-amino-éthyl-phosphonique;
l'acide N-(7-fluoro-2,3-dioxo-1,2,3,4-tétrahydro-quinoxaline-5-ylméthyl)-α-amino-éthylphosphonique
l'acide P-benzyl-N-(2,3-dioxo-7-nitro-1,2,3,4-tétra-hydroquinoxaline-5-ylméthyl)-amino-méthylphosphinique ;
l'acide P-méthyl-N-(2,3-dioxo-7-nitro-1,2,3,4-tétra-hydroquinoxaline-5-ylméthyl)-α-aminoéthyl-phosphinique;
l'acide P-benzyl-(7-nitro-2,3-dioxo-1,2,3,4-tétra-hydroquinoxaline-5-yl)-amino-méthane-phosphinique ;
l'acide P-méthyl-(7-nitro-2,3-dioxo-1,2,3,4-tétra-hydroquinoxaline-5-yl)-amino-méthane-phosphinique ;
l'acide N-(7-nitro-2,3-dioxo-1,2,3,4-tétrahydro-quinoxaline-5-ylméthyl)-4-phosphono-butyrique-amide ;
l'acide N-(7-nitro-2,3-dioxo-1,2,3,4-tétrahydro-quinoxaline-5-ylméthyl)-2-phosphono-acétique-amide ;
l'acide N-(7-nitro-2,3-dioxo-1,2,3,4-tétrahydro-quinoxaline-5-ylméthyl)-3-phosphono-propénique-amide ;
l'acide N-(7-nitro-2,3-dioxo-1,2,3,4-tétrahydro-quinoxaline-5-ylméthyl)-2-phosphono-indane-2-carboxylique-amide ;
l'acide N-benzyl-N-(2,3-dioxo-7-nitro-1,2,3,4-tétra-hydroquinoxaline-5-ylméthyl)-amino-méthyl-phosphonique ;
l'acide N-benzyl-N-(7-bromo-2,3-dioxo-1,2,3,4-tétra-hydroquinoxaline-5-ylméthyl)-amino-méthyl-phosphonique ;
l'acide N-benzyl-N-(7-chloro-2,3-dioxo-1,2,3,4-tétrahydroquinoxaline-5-ylméthyl)-amino-méthyl-phosphonique ;
l'acide N-benzyl-N-(2,3-dioxo-7-fluoro-1,2,3,4-tétrahydroquinoxaline-5-ylméthyl)-amino-méthyl-phosphonique ;
l'acide N-benzyl-N-(2,3-dioxo-7-nitro-1,2,3,4-tétra-hydroquinoxaline-5-ylméthyl)-α-amino-éthane-phosphonique ;
l'acide N-(2,3-dioxo-7-fluoro-1,2,3,4-tétrahydro-quinoxaline-5-ylméthyl)-α-amino-éthyl-phosphonique;
l'acide N-(2,3-dioxo-7-nitro-1,2,3,4-tétrahydro-quinoxaline-5-ylméthyl)-α-amino-propyl-phosphonique ;
l'acide N-(2,3-dioxo-7-nitro-1,2,3,4-tétrahydro-quinoxaline-5-ylméthyl)-α-amino-(tétrahydropyrane-4-yl)-phosphonique ;
l'acide N-(2,3-dioxo-7-nitro-1,2,3,4-tétrahydro-quinoxaline-5-ylméthyl)-α-amino-(pipéridine-4-yl)-phosphonique ;
l'acide N-(2,3-dioxo-7-nitro-1,2,3,4-tétrahydro-quinoxaline-5-ylméthyl)-α-amino-(furane-2-ylméthyl)-phosphonique ;
l'acide N-(2,3-dioxo-7-nitro-1,2,3,4-tétrahydro-quinoxaline-5-ylméthyl)-α-amino-(2-méthoxy)-éthyl-phosphonique ;
l'acide N-(2,3-dioxo-7-nitro-1,2,3,4-tétrahydro-quinoxaline-5-ylméthyl)-2-amino-2-cyclohexyl-éthyl-phosphonique ;
l'acide N-(2,3-dioxo-7-nitro-1,2,3,4-tétrahydro-quinoxaline-5-ylméthyl)-β-amino-isopropyl-phosphonique;
l'acide -N-(2,3-dioxo-7-nitro-1,2,3,4-tétrahydro-quinoxaline-5-ylméthyl)-2-amino-cyclohexyl-phosphonique ;
l'acide N-(2,3-dioxo-7-nitro-1,2,3,4-tétrahydro-quinoxaline-5-ylméthyl)-2-amino-2-méthyl-propyl-phosphonique ;
l'acide N-(2,3-dioxo-7-nitro-1,2,3,4-tétrahydro-quinoxaline-5-ylméthyl)-2-amino-butyl-phosphonique ;
l'acide N-(2,3-dioxo-7-nitro-1,2,3,4-tétrahydro-quinoxaline-5-ylméthyl)-2-amino-heptyl-phosphonique ;
l'acide N-(2,3-dioxo-7-nitro-1,2,3,4-tétrahydro-quinoxaline-5-ylméthyl)-2-amino-3-phényloxy-propyl-phosphonique ;
l'acide N-(2,3-dioxo-7-nitro-1,2,3,4-tétrahydro-quinoxaline-5-ylméthyl)-2-amino-2-p-tolyl-éthyl-phosphonique ;
l'acide N-(2,3-dioxo-7-nitro-1,2,3,4-tétrahydro-quinoxaline-5-ylméthyl)-2-amino-2-(2-méthoxy-phényl)-éthyl-phosphonique;
l'acide N-(2,3-dioxo-7-nitro-1,2,3,4-tétrahydro-quinoxaline-5-ylméthyl)-2-amino-2-(4-fluoro-phényl)-éthyl-phosphonique ;
l'acide N-(2,3-dioxo-7-nitro-1,2,3,4-tétrahydro-quinoxaline-5-ylméthyl)-2-amino-2-phényl-éthyl-phosphonique ;
l'acide N-(2,3-dioxo-7-nitro-1,2,3,4-tétrahydro-quinoxaline-5-ylméthyl)-2-amino-1-(furane-2-yl)-éthyl-phosphonique ;
l'acide N-(2,3-dioxo-7-nitro-1,2,3,4-tétrahydro-quinoxaline-5-ylméthyl)-2-amino-1-(4-fluoro-phényl)-éthyl-phosphonique;
l'acide N-(2,3-dioxo-7-nitro-1,2,3,4-tétrahydro-quinoxaline-5-ylméthyl)-2-amino-1-(4-méthoxy-phényl)-éthyl-phosphonique ;
l'acide N-(2,3-dioxo-7-nitro-1,2,3,4-tétrahydro-quinoxaline-5-ylméthyl)-2-amino-1-(3-méthoxy-phényl)-éthyl-phosphonique ;
l'acide N-(2,3-dioxo-7-nitro-1,2,3,4-tétrahydro-quinoxaline-5-ylméthyl)-2-amino-1-(2-chloro-phényl)-éthyl-phosphonique ;
l'acide N-(2,3-dioxo-7-nitro-1,2,3,4-tétrahydro-quinoxaline-5-ylméthyl)-2-amino-1-(4-tolyl)-éthyl-phosphonique ;
l'acide N-benzyl-N-(7-bromo-2,3-dioxo-1,2,3,4-tétra-hydroquinoxaline-5-ylméthyl)-α-amino-éthyl-phosphonique ;
l'acide N-benzyl-N-(7-chloro-2,3-dioxo-1,2,3,4-tétrahydroquinoxaline-5-ylméthyl)-α-amino-éthyl-phosphonique et
l'acide N-benzyl-N-(2,3-dioxo-7-fluoro-1,2,3,4-tétrahydroquinoxaline-5-ylméthyl)-α-amino-éthyl-phosphonique ;
et leurs sels.

2. Composé selon la revendication 1 sous une forme libre ou sous forme d'un sel pharmaceutiquement utilisable dans un procédé de traitement thérapeutique de l'organisme humain ou animal.

3. Préparation pharmaceutique, contenant un composé selon la revendication 1 ou un sel de celui-ci pharmaceutiquement utilisable en plus des adjuvants et véhicules pharmaceutiques habituels.

4. Utilisation d'un composé selon la revendication 1, pour la production d'un médicament pour le traitement des états pathologiques qui répondent à un blocage des récepteurs excitatoires des acides aminés.

5. Utilisation d'un composé selon la revendication 1 pour là production d'un médicament pour le traitement des maladies neurodégénératives, des affections cérébrales ischémiques, des spasmes vasculaires et musculaires, des convulsions et des angoisses et états douloureux.

6. Utilisation selon la revendication 5, pour le traitement de l'épilepsie.
